# EUROPEAN PATENT SPECIFICATION

(11) **EP 0 987 036 B1**
(45) Date of publication and mention of the grant of the patent: **12.04.2006**
(21) Application number: 99123932.8
(22) Date of filing: 20.05.1998
(51) Int. Cl.: G01N 21/17, A61M 1/36

(54) **Optical sensors for cell processing systems**
Optische Sensoren für Zellbehandlungssysteme
Capteurs optiques pour systèmes de traitement de cellules

(30) Priority: 20.05.1997 US 47213 P
(43) Date of publication of application: 22.03.2000
(62) Divisional of application: 98924826.5
(73) Proprietor: Zymequest, Inc., Beverly, MA 01915-6122 (US)
(72) Inventor: Jorgensen, Glen, Marlboro, MA 01752 (US); Barry, Donald, Norwood, MA 02062 (US); Edwards, Bruce H., Marlboro, MA 01752 (US); Fennelly, Jeremy, Ashland, MA 01721 (US); O'Brien, John P., Brighton, MA 02135 (US); Sacco, Victor Jr., Revere, MA 02151 (US); Martin, Roy E., III, Worcester, MA 01609 (US); Susser, Mark, Weston, MA 02193 (US)
(74) Representative: Murphy, Colm Damien

(56) References cited:
- EP-A- 0 682 953
- WO-A-96/40322
- US-A- 4 049 192

## Description

The present invention relates to an optical sensor for a cell processing system and to a method for characterising a sterile fluid during processing of biological cells in such a cell processing system.

Cell processing includes steps where cells or cell elements are treated with different process chemicals or are washed and then separated from a liquid phase. For example, when preparing frozen erythrocytes for transfusion, erythrocytes are separated from cryopreservatives and other blood components such as white cells, platelets and sub-cellular debris. The entire process must be performed under sterile conditions that minimize the risk of contamination. Furthermore, whole blood is separated into its various therapeutic components such as red blood cells, white blood cells, platelets and plasma which are later transfused. There are different cell processing systems that process biological cells in an automated or semi-automated way. These systems may use a controller connected to various sensors and valves for controlling the process and helping an operator to maximize the processing efficiency. However, these systems do not interactively adjust the process based on the amount or type of the processed cells or different processing conditions.

Hospitals require a constant blood supply for transfusions. After donors provide blood, regional blood centers are responsible for ABO typing, infectious disease testing, component manufacturing, and distribution of red blood cells to hospitals. The hospitals again, test the A. B, AB, O blood group and cross match the available blood units to the appropriate patients. Since group O blood can be transfused universally, there is a high demand for group O blood, in general, and especially in emergency situations where the delay caused by typing and matching is not acceptable. Furthermore, the processed blood has a relatively short shelf life of 42 days, after which it may not be transfused. The balancing of the inventory of red blood cells is extraordinarily complex. On a daily basis, the regional blood centers must match the demand for different blood groups with the available supply held at the blood centers, and at its hospital customer sites around the country. The individual blood units are constantly moved within the system in order to match daily variation in supply and demand. In fact, individual units are frequently moved three to four times within the system before finally being transfused. Even with the best efforts by the participants to ensure that each collected unit is ultimately transfused, 4% to 8% of all collected units outdate before transfusion and must be discarded. A processing system that would reproducibly convert A, B, or AB type blood to O type blood would satisfy a crucial need in this field. The availability of O blood cells would improve red cell availability, substantially eliminate red cell outdating caused by the inability to match units with recipients within the 42 day outdate window, eliminate the need for the frequent reshipment of blood units in order to match the daily supply and demand, and eliminate the need for retesting for the blood type.

EP-A-0682953 discloses apparatus for washing salvaged blood comprising a reservoir for collecting blood lost from the wound of a patient, a centrifuge and a waste container. Blood is pumped from the reservoir into the centrifuge by means of a pump, and waste washed from the salvaged blood is collected in the waste container via an effluent line. The effluent line passes through an optical line sensor which senses the turbidity of fluid flowing through the effluent line and controls *inter alia* the speed of the pump. The line sensor includes a housing having a groove through which the effluent line passes. Two LED-detector units straddling the effluent line are housed within the housing. Each LED-detector unit comprises an LED for emitting light and a detector for detecting light emitted by the corresponding LED, One of the LEDs emits light of a wavelength of 670 nm (red light), and the other emits light of a wavelength of 565 nm (yellow-green light). The LEDs are located on opposite sides of the effluent line from the corresponding detectors, and light from the LEDs passes through the effluent line to be detected by the respective detectors. The yellow-green light emitted by said other LED is absorbed by free haemoglobin and absorbed and scattered by red blood cells, whilst the red light emitted by said one LED is absorbed and scattered by red blood cells, but is not readily absorbed by free haemoglobin. Owing to the different wavelengths of light employed for the two LED detector units, the other LED detector unit is sensitive to detecting small amounts of free haemoglobin, whilst the one LED detector unit is relatively insensitive to free haemoglobin levels. Both LED detector units detect red blood cells and cell stroma in the effluent line.

WO-A-96/40322 discloses apparatus for extracorporeal blood processing which includes an extracorporeal tubing circuit comprising a plurality of tubing assemblies and a cassette assembly to which the tubing assemblies are connected. The tubing assemblies include a blood inlet/blood component tubing sub-assembly which provides an interface between the cassette assembly and a blood processing vessel. The blood input/blood component tubing assembly comprises platelet outlet tubing which includes a cuvette for use in the detection of red blood cells (via an interfacing red blood cell spillover detector provided on a blood component separation device).

According to one aspect of the present invention there is provided an optical sensor as claimed in claim 1 below.

The optical sensor may include one or several of the following features:

The light source and the light detector may be arranged in a transmission geometry. The light source, the light detector and the control circuit may be enclosed in a housing. The housing may be suitable for sterilization by gama rays or by other means.

The fluid distribution manifold may include an integral component constructed to be placed in close proximity to the housing and arranged to define uniquely the position of the cuvette relative to the light source and the light detector.

The cuvette may be made of an optical material suitable for sterilisation. The cuvette may be made of an optical material suitable for sterilisation using gama radiation. The cuvette and the fluid distribution manifold may be disposable.

The light source and the light detector may be located in a sealed housing constructed and arranged for wet cleaning.

The light source may include a light emitting diode (LED) constructed and arranged to emit light of about 560 nm and about 640 nm. The light detector may include a silicon diode. The diode sensor may be arranged to detect red blood cells in the fluid.

The control circuit may be constructed to activate the light source and the light detector to perform repeated measurements over a short period of time to increase precision of the characterisation. The control circuit may be constructed to calibrate operation of the optical sensor after placement of the cuvette.

The optical sensor may be further arranged to provide data to the control module to actuate re-distribution of fluids flowing in the conduits.

In accordance with another aspect of the present invention there is provided a method of characterising a sterile fluid as claimed in claim 6 below.

The method may include further processing biological cells in a processing module by employing process chemicals including saline; expressing the process chemical from the processing module; transferring a flow the expressed process chemicals to a waste container via conduits, wherein at least one of the conduits is permanently connected to and in communication with the cuvette. The method may also include emitting light of at least one selected wavelength generated by a light source; detecting light that was emitted from the source and has interacted with the fluid flowing in side the cuvette; characterising the fluid in the cuvette based on the detected light; and upon detecting presence of the processed biological cells during the characterising, redirecting the flow to transfer the processed biological cells to a cell container.

The emitting may include generating light of about 560 nm and about 640 nm. The redirecting may include temporarily reversing the expressing action performed by the processing module to draw back the detected biological cells, and actuating a valve by the control circuit. The biological cells may include red blood cells.

Following is a description by way of example with reference to the accompanying drawings of embodiments of the present invention.

In the drawings:
Fig. 1 is a perspective view of an interactive cell processing system.
Fig. 2 is a conceptual flow diagram displaying operation of an interactive cell processing system.
Fig. 3 is a block diagram of the interactive cell processing system of Fig. 1.
Figs. 4 and 4A show a flow diagram of a process for enzymatic conversion of red blood cells.
Fig.5 is a perspective view of an optical sensor used in the cell processing system of Fig. 1.
Fig. 6 is a schematic diagram of the elements used in optical sensor of Fig. 5.
Fig. 7 is a perspective view of a fluid distribution module including a partial view the optical sensor of Fig. 5.
Fig. 8 is a partially exploded view of the fluid distribution module of Fig. 7 with another view of the optical sensor of Fig. 5.
Fig. 9 is a further exploded view of the fluid distribution module of Fig. 6, showing a -pump valve assembly, housing, fluid distribution manifold, connector, and spring knobs of Fig. 8.
Fig. 10 is a front plan view of the distribution manifold of Figs. 7-9, and a schematic view of a pump and filter.
Fig. 10A is an exploded view of the distribution manifold and filter of Fig. 10.
Fig. 11 is an exploded view of the distribution manifold and connector of Figs. 7-10.
Fig. 12 is a rear plan view of the front plate of the distribution manifold of Fig. 11.
Fig. 13 is a rear plan view of the membrane of the distribution manifold of Fig. 11.
Fig. 14 is a cross-sectional view of Fig. 8, taken along lines 12 -12.
Figs. 15 and 16 are front and top plan views, respectively, of the connector of Figs. 7-9.
Fig. 17 is a perspective view of a multi-compartment bag connected by tubing to the connector of Figs. 15 and 16.
Fig. 18 is a left side perspective view of the Fig. 1 system.
Fig. 19 is an isometric exploded view of components of a subassembly used for expressing selected fluid materials disposed in a flexible container.
Fig. 20 is an isometric exploded view of certain of the components shown in Fig. 19
Fig. 21 is a side cross-sectional view of certain components of the expressor system subassembly shown in Fig. 19 taken along one plane which does not intersect one of the fluid flow grooves 410 in chuck 408.
Fig. 22 is another side cross-sectional view of certain components of the expressor system subassembly shown in Fig. 19 taken along a plane which does intersect one of the fluid flow grooves 410 in chuck 408.
Fig. 23 is a schematic side cross-sectional view of the Fig. 22 view showing the flexible membrane component 411 seated initially at the beginning of a processing cycle along the curved surface of the bowl or donut shaped separation chamber 421 of chuck 408.
Fig. 24 is a close-up side cross-sectional view of a portion of Fig. 23 showing the expressor fluid chamber 420 partially filled with expressor fluid at a later stage in a typical processing cycle.
Fig. 25 is a view of Fig. 24 at an even later stage of a typical processing cycle showing the expressor fluid chamber 42 filled to a greater degree/volume than the chamber is filled in Fig. 24.
Fig. 26 is another schematic side cross-sectional view of the expressor system subassembly of Figs. 18-25 showing additional components by which expressor fluid is input from a pumping source through a central drive shaft which is rotatably driven.
Fig. 27 is an isometric view of the Fig. 19 components in assembled form.
Fig. 28 is an exploded isometric view of a rotating seal used in conjunction with an expressor system.
Fig. 29 is an isometric view of the Fig. 28 components in assembled form.
Fig. 30 is an exploded side cross-sectional view of the Fig. 28 rotating seal components.
Fig. 31 is a cross-section view of the rotating seal apparatus.
Fig. 32 is an exploded top perspective view of the rotating seal apparatus and processing container.
Fig. 33 is an exploded bottom perspective view of the rotating seal apparatus.

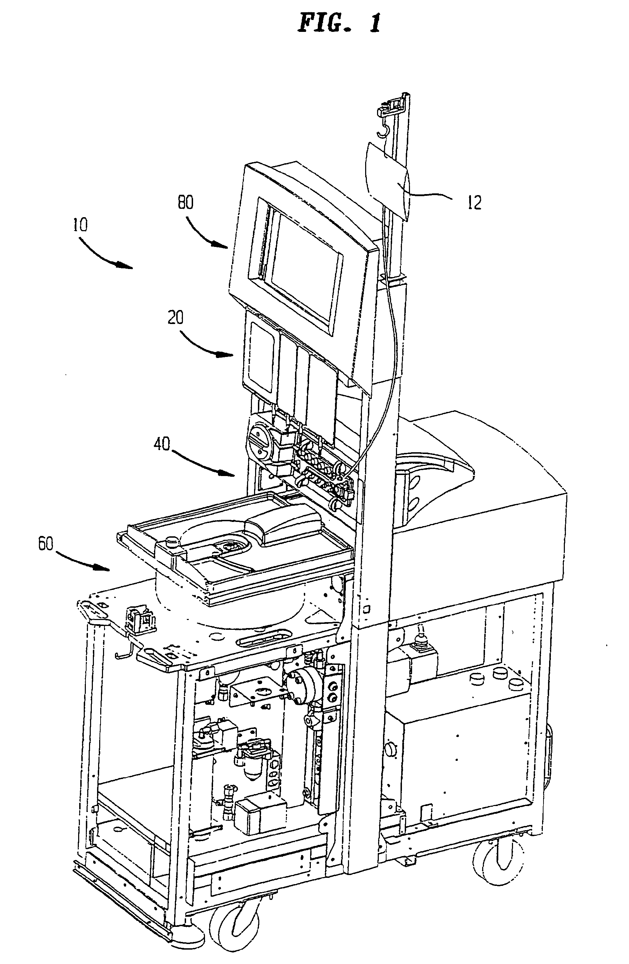

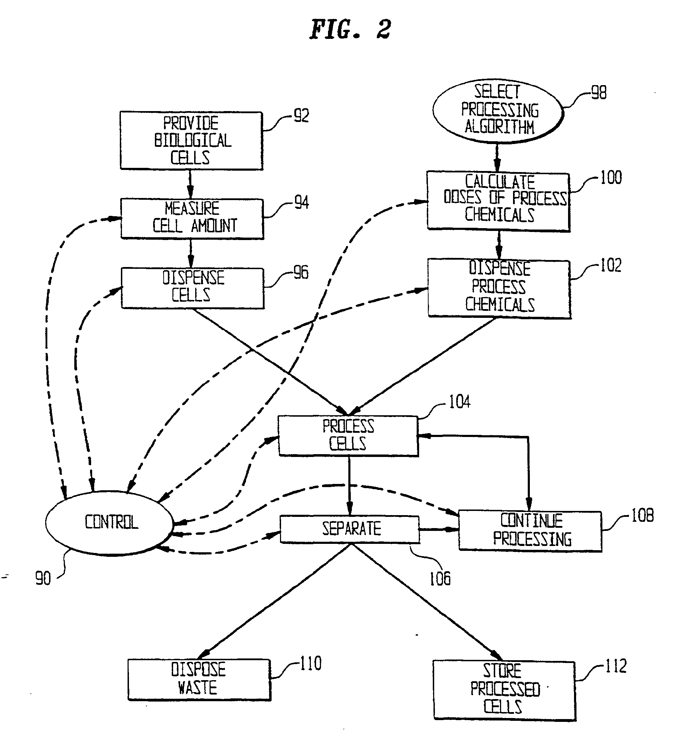

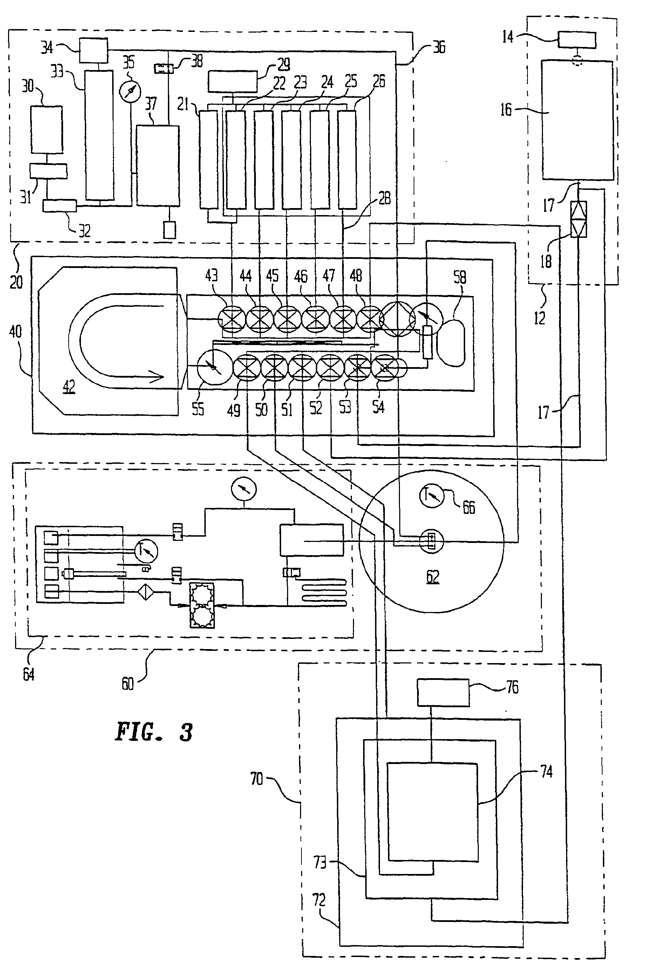

Referring to Figs. 1 and 3, an interactive cell processing system 10 includes a cell module 12, a supply module 20, a fluid distribution module 40, a processing module 60, a collection module 70 (not shown in Fig. 1) and a control module 80. These modules are operatively interconnected for processing biological cells in a sterile environment. Cell module 12 is constructed for a short term or long term storage of biological cells for processing. Supply module 20 includes several containers for storing different process chemicals including saline, or other fluids used for washing the processed cells and also includes sterile air. The containers are connected to fluid distribution module 40 by a set of conduits. Fluid distribution module 40 includes several valves and sensors for dispensing controlled amounts of the process chemicals from supply module 20 to processing module 60 and for dispensing a known amount of the biological cells from cell module 12 to processing module 60. Furthermore, fluid distribution module 40 is constructed to direct the process waste from processing module 60 to a waste container 72 and the processed cells to a cell storage container 74, both of which are located in collection module 70, while maintaining the purity and sterility of the cells. Control module 80 directs the entire process according to a selected algorithm.

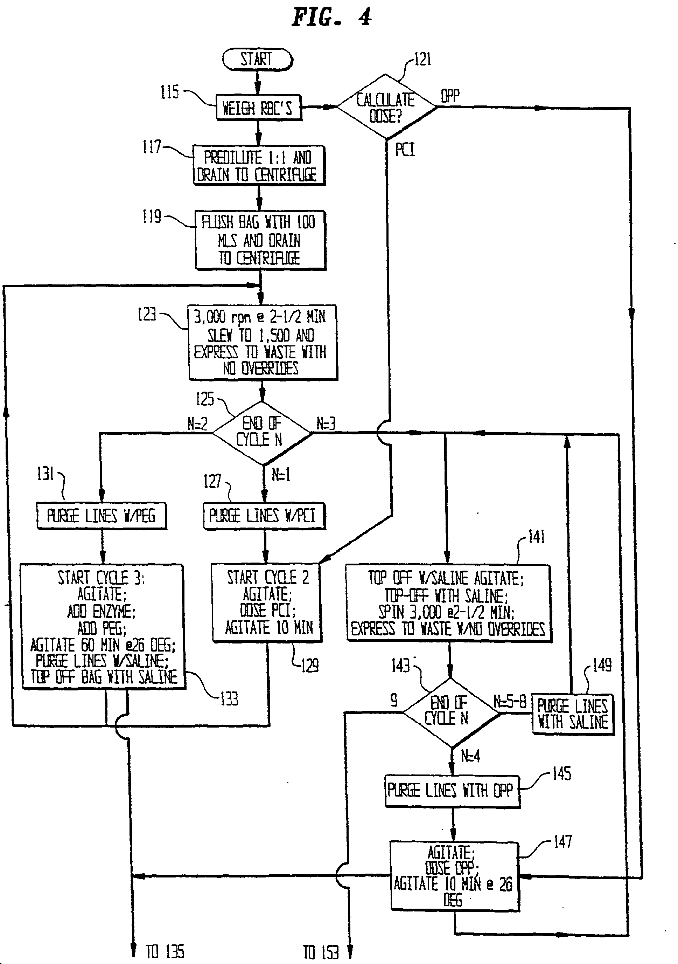

In general, the operation of cell processing system 10 is shown in Fig. 2. Control module 80 executes a processing algorithm selected initially (98). Control module 80 includes a logic controller that receives real-time data from several in-line sensors arranged in a processing loop. A mass sensor (or a volume sensor) measures an initial amount of the provided biological cells (94) and sends the data to control module 80. Control module 80 controls the amount of cells dispensed to processing module 60 in accordance with the processing algorithm. Based on the provided amount of the biological cells, control module 80 also calculates the individual doses of the process chemicals (100) and directs a set of control valves to dispense the chemicals (102) in a selected order to processing module 60, again in accordance with the processing algorithm.

Control module 80 executes iteratively the processing algorithm. Control module 80 receives data from the individual sensors (e.g., a weight sensor, a volume sensor, a temperature sensor, an optical sensor, a resistance or capacitance sensor, a flow sensor, a pressure sensor or another sensor arranged to monitor the transferred matter in a liquid, gaseous or solid state). After dispensing the selected amount of one or several processing chemicals to processing module 60, control module 80 regulates the temperature and the time of processing and directs the processing module to agitate, mix or otherwise treat the cells with the process chemicals. Depending on the processing algorithm, control module 80 may manage one or several processing cycles. At the end of each cycle, processing module 60 may separate the processed cells from intermediate products and from the process waste. During the separation process, fluid distribution module 40 detects the fluid component being expressed from processing module 60 and directs the separated components to different containers for disposal (110) or for storage (112). Each processing cycle may use a different processing chemical and different processing conditions. Cell processing system 10 can also process different types of cells at the same time or sequentially. Furthermore, cell processing system 10 may also partially process biological cells and then store them in cell storage container 74 (shown in Fig. 3), which may include a temperature control system. The processed cells may be later automatically dispensed from cell storage container 74 and processed using another processing algorithm. The processed cells may also be grown in culture prior to another use.

Based on the starting weight of the biological cells, the controller calculates the dosage of the processing chemicals. Supply module 20 includes a weight sensor 29 for providing the weight of each process chemical to the controller. During the process, the controller confirms that correct amount of each process chemical has been transferred by measuring the change in the weight of the process chemical stored in supply module 20 and the initial weight of the chemical. The process chemicals in a fluid state are pumped through a 0.2 micron filter to assure sterility. A pressure transducer is mounted up-stream from the filter. If the fluids being pumped through the filter have a variable viscosity, the controller will adjust the pumping speed to yield a constant pressure drop across the filter membrane.

Processing module 60 is designed to assure identical processing conditions (e.g.. pressure, temperature, mixing, processing time or other) for large and small amounts of the biological cells provided for processing. For this purpose, processing module 60 includes a processing chamber that has a variable volume design. Depending on the volume of the processed cells and other processing chemicals transferred into the processing chamber, the controller changes the chamber volume. The volume change is achieved by a movable wall that may be a membrane. Processing module 60 includes another pressure sensor for measuring the pressure inside the processing chamber and also includes a temperature sensor for measuring the temperature inside the processing chamber. Based on the data from the temperature sensor, a heat transfer system can provide or remove heat from the processing chamber.

Cell processing system 10 may process or separate cells and/or cell elements from different liquids or solids. Such cells and cell elements include, but are not limited to, erythrocytes (*i.e*., red blood cells); leukocytes (*i.e.,* white blood cells, including lymphocytes, granulocytes, and monocytes); blood cell progenitors (*e.g*., primitive stem cells, burst forming units, reticulocytes, megakaryocytes, etc.); cell fragments (*e.g*., platelets, subcellular elements such as nuclei, debris, etc.); epithelial cells; endothelial cells; mesothelial cells; cells -of normal tissues (*e.g*., liver cells, kidney cells, bladder cells, lung cells, pancreatic cells, embryonic cells, fetal cells, etc.); cells of abnormal tissues (*e.g*., malignant cells), and other.

Referring again to Fig. 3, cell module 12 includes a weight sensor 14 arranged to weigh red blood cells provided in a PVC bag 16. Tubing 17 connects bag 16 to a leuko filter 18 and to fluid distribution module 40. Supply module 20 includes a bag 21 with enzyme A1/B, a bag 22 with enzyme A2, a bag 23 with 140 mMolar potassium phosphate dibasic (DPP), a bag 24 with polyethylene glycol (PEG), a bag 25 with storage solution, and a bag 26 with phosphate citrate isotonic (PCI). Bags 22, 23, ..., 26 are made of cryovac M312. Each bag is connected by tubing 28 to fluid distribution module 40. Weight sensor 29 is constructed to weigh any of the above-mentioned fluids located in supply module 20. Supply module 20 also includes a compressor 30 connected via a filter 31 and a check valve 32 to air reservoir 33, which stores sterile air used for cell processing. Pressure switch and sensor 34 is in communication with air tubing 36, which delivers sterile air to an air filter located in fluid distribution module 40. A regulator 37, connected to a solenoid valve 36, regulates the air pressure provided to fluid distribution module 40 and to processing module 60. Fluid distribution module 40 includes a peristaltic pump 42, and twelve plunger valves 43, 44,..., and 54 connected to a set of conduits for distributing the process chemicals and the cells during the automated process. The logic controller can close or open any combination of the twelve valves to redirect the fluid flowing inside the conduits. A pressure sensor 55 measures the fluid pressure during the process, and a optical detector 58 monitors the fluid to and from processing module 60. Processing module 60 includes a centrifuge 62 and an expressor system 64. An IR temperature sensor 68 monitors the temperature of the process chemicals or the cells located inside centrifuge 62. Collection module 70 includes a waste bag 72, a saline solution bag 74, and a product bag 76. Collection module 70 also includes a weight sensor 76 connected to product bag 76 and arranged to weigh the processed red blood cells.

The controller controls the volume of the processing chamber of centrifuge 62 to assure identical processing conditions for large or small amounts of the red blood cells. The processing chamber includes a flexible wall for containing expressor fluid inside the processing chamber. For small volumes, expressor system 64 pumps expressor fluid into the chamber until the pressure transducer at the chamber signals a full condition. This pre-filling step assures that different amounts of red blood cells are subjected to the same accumulated centrifugal force and mechanical stresses due to packing. Otherwise, smaller amounts would spin longer and pack harder as the expressor fluid fills the processing chamber during the expression step.

During the process, the controller receives input from IR temperature sensor 66, which measures the temperature of the red blood cells. If the temperature is less than the set point, expressor of system 64 increases the temperature of the expressor fluid. Conversely, if the temperature is greater than the set point, expressor of system 64 decreases the temperature of the expressor fluid. A control loop continuously monitors the temperature of the processed cells.

Processing module 60 also includes a second pressure transducer that monitors the pressure of the sterile air on the rotating seal. If the seal is working, this pressure only fluctuates slightly between established limits. If the pressure drops below the established threshold, a warning condition is initiated that calls for a check of the rotating seal as well as other possible causes of failure.

Expressor fluid system 64 included a third pressure transducer that measures the pressure of the expressor fluid which is an indirect measure of the pressure on the red blood cells. The controller adjusts the expressor pump speed to assure that pressure is within accepted limits and cells are protected from damage. If the pressure is too low, the pump rate is increased to speed up the expression cycle. If the pressure is too high, the pump is slowed down to protect the cells from excessive pressure. This also protects the seal from excessive pressure as well.

Optical sensor 58 sensor monitors the color and the turbidity of the transferred fluids. Specitically, optical sensor 58 also monitors the supernatant expressed from the centrifuge chamber. When red cells are detected in the supernatant, the controller responds by stopping the expressor pump to avoid losing any cells to waste or responds by switching valves to collect the cells in a separate storage bag depending on which cycle is being performed.

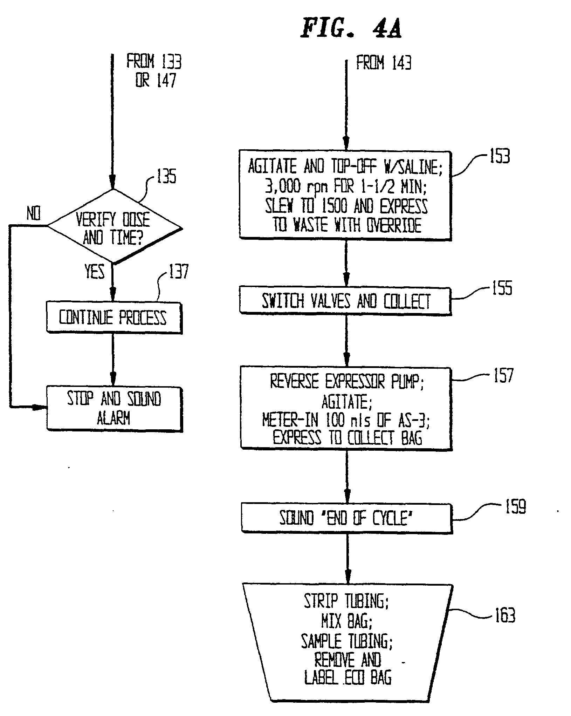

Referring to Figs. 4 and 4A. the cell processing system of Fig. 3 is used for enzymatic conversion of red blood cells to type O red blood cells. The enzymatic conversion process starts in step 115 by weighting the provided amount of red blood cells. In step 117, based on the starting weight of the provided red blood cells, the system dilutes the red blood cells dispensed to the processing bag located inside centrifuge 62, shown in Fig. 3, with saline in the 1:1 ratio,and also flushes the bag with 100 ml of saline (step 119). In step 121, the controller calculates the correct dosage of PCI to obtain the ratio of 65 ml of PCl for 100 ml of red blood cells. The controller also calculates the correct dosage of DPP to obtain the ratio of 110 ml of DPP for 100 mls of red blood cells. Prior to executing step 123, the controller confirms that the correct amount of saline was transferred to centrifuge 62. In step 123, the centrifuge spins at 3000 RPM for about 2.5 minutes and then slows down to about 1500 RPM and expresses the saline waste while the washed red blood cells are left in the processing bag.

Next, in step 127, the system purges the tubing with PCI and dispenses the dose calculated in step 121, of PCI to the processing bag. PCI (Phosphate Citrate Isotonic) includes citric acid monohydrate 10.7 g/L, sodium phosphate dibasic (anhydrous) 2.7 g/L, sodium chloride 6.4 g/L suspended in one liter of sterile water having pH = 2.8 ± 0.05. The required dose is 65 mls of 2.8 pH PCI Buffer for every 100 mls of the 85 crit cell mass. In step 129, the centrifuge thoroughly mixes the solution during addition of PCI and them occasionally agitates the red blood cells and PCI mixture for about 10 minutes for equilibration to reduce the pH of the packed red blood cells from approx 7.0 to 5.5. Then, in step 123, the centrifuge expresses the separated waste (also called supernatant) while the red blood cells are left in the processing bag.

In step 131, the system purges the tubing with PEG and dispenses the calculated dose to the processing bag. In step 133, the system also adds enzymes to the processing bag, based on the amount of red blood cells measured in step 115. The enzyme includes 12.5 ml of rB-zyme or 25 ml of a suspension of exo- and endo- rA-zyme and the PEG dose is 23 ml per 250 ml of 85 crit cell suspension. The centrifuge agitates for 60 minutes at the incubation temperature of 26°C for rB-zyme and at 37°C for rA-zymes. The enzyme is suspended in 5.5 pH PCI Buffer, PEG is 1450 MW suspended in 5.5 pH PCI. The system also verifies the dose, the time and the temperature according to the algorithm (step 135) and continues the red blood cells conversion if all parameters are satisfied. Then, the system purges the tubing with saline and fills up the processing bag with saline. In step 123, the centrifuge spins the solution at 3000 RPM for about 2.5 minutes and then slows down to about 1500 RPM and expresses the supernatant waste while the washed red blood cells are left in the processing bag.

After the red blood cell conversion, the centrifuge expresses the supernatant (step 123). Next, in step 141, the system dispenses saline to the processing bag, agitates the mixture and spins the mixture at about 3000 RPM for about 2.5 minutes. The centrifuge expresses the waste, and the system restores the 85 crit cell mass. In step 145, purges the tubing with DPP to restore subsequently pH of converted red blood cells. In step 147, the system dispenses DPP by metering 110 ml of DPP Buffer for every 100 ml of the 85 cri cell suspension. The system dispenses 140 mM potassium phosphate dibasic with pH 9.0 ± 0.1 (DPP) that includes potassium phosphate dibasic (anhydrous) 24.4 g/L suspended in one liter of sterile water. The centrifuge mixes thoroughly the liquid during addition of the buffer and equilibriates at 26° C for 10 minutes also mixing occasionally during the equilibration. Next, in step 141, the system fills the processing bag with saline, agitates the mixture, and expresses the waste while the red blood cells are left in the processing bag.

Next, this system purges the lines with saline and washes the red blood cells several times by filling the processing bag with saline and subsequently expressing the waste (steps 141, 143 and 149). These steps remove the residual buffer, enzyme, PEG and phosphate to a level approximately equivalent to 99.9999%. After expressing the used saline in the last washing cycle (step 153), the system restores the 85 crit cell mass.

The controller directs fluid distribution module 40 to switch the tubing to collect the processed red blood cells in storage bag 74. This process is controlled by optical detector 58 (shown in Fig. 3). After the optical detector detects red blood cells, in step 155, the expressor pump reverses its pumping direction to draw back into the processing bag the red blood cells from the tubing located between the processing bag and the optical detector. This is done to minimize the loss of red blood cells. Then, fluid distribution system 40 redirects the expressed red blood cells to storage bag 74. When the processing cycle is completed (step 157) the controller meters 100 mls of nutracell storage solution for 250 ml of the 85 crit cell suspension. This solution is then stored in the storage bag made from a material approved for 42-day storage (step 163).

The cell processing system may be used for enzymatically converting blood type as described, for example, in United States Patents 4,330,619, 4,427,777 and 4,609,627 to Goldstein.

Optical sensor 58 sensor monitors the color and the turbidity of the transferred fluids. Specifically, optical sensor 58 also monitors the supernatant expressed from the centrifuge chamber. In step 153, when red cells are detected in the supernatant, the controller responds by stopping the expressor pump to avoid losing any cells to waste. In step 155, the controller switches valves to collect the cells in cell storage container 74.

Optical sensor 58 is constructed and arranged to optically characterize a fluid being transferred within fluid distribution system 40. Since the processed cells must be maintained in a sterile environment during the entire process, the optical sensor has to satisfy the corresponding requirements. The requirements include a sterile and easily replaceable optical chamber. The entire design is waterproof and enables easy sterilization of all outside surfaces in accordance with the corresponding regulations.

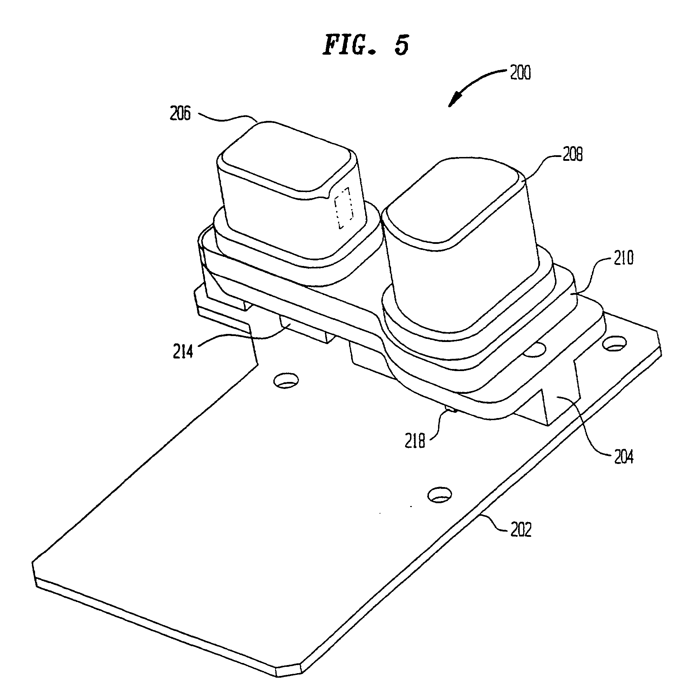

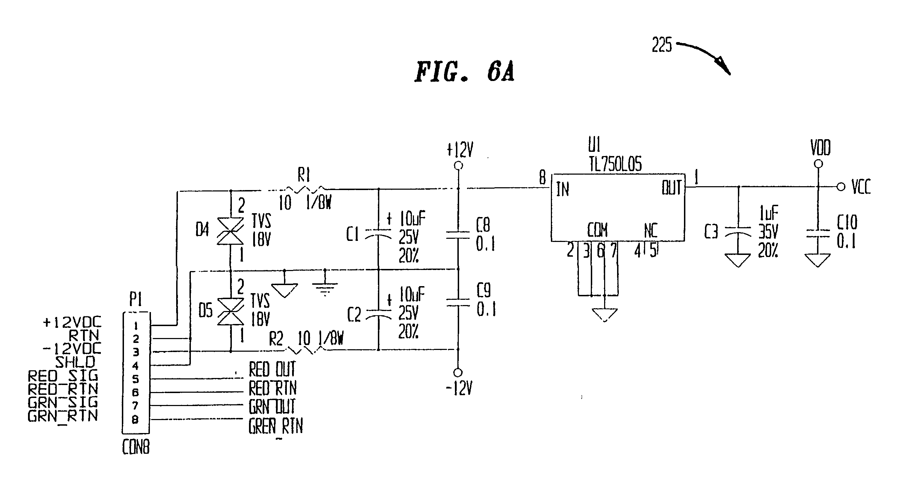

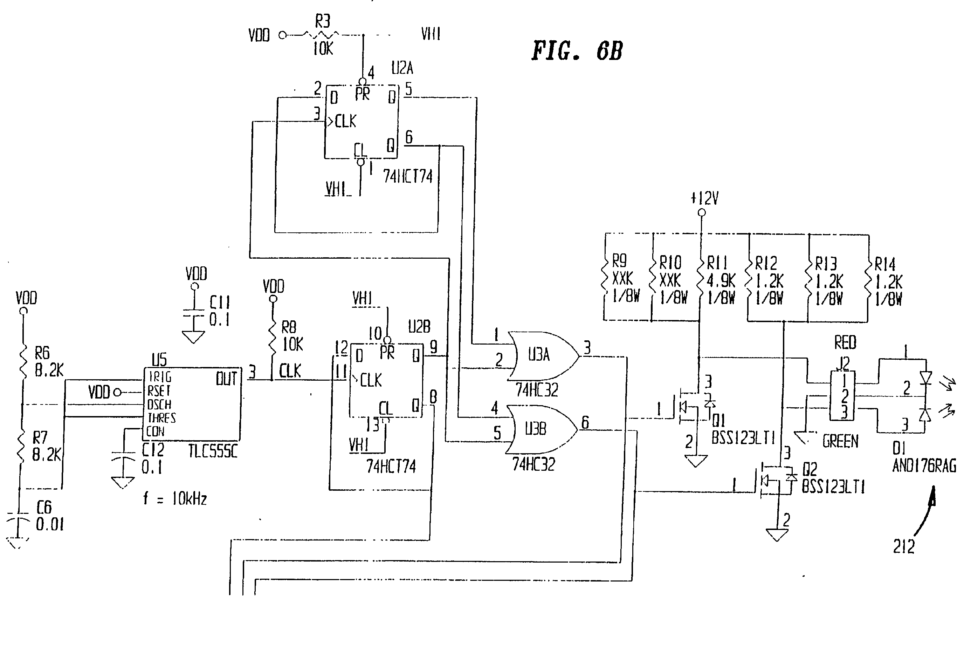

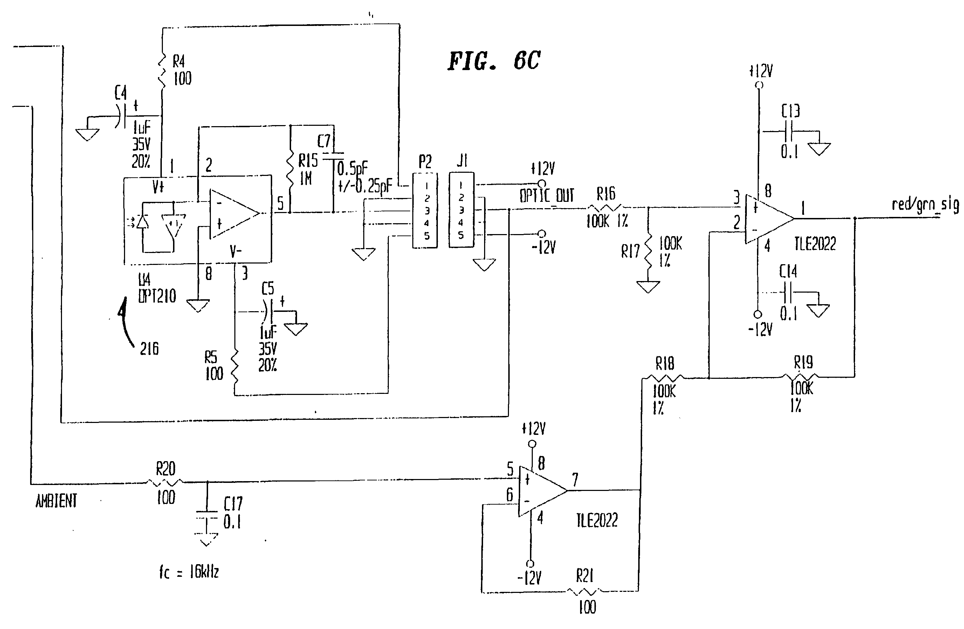

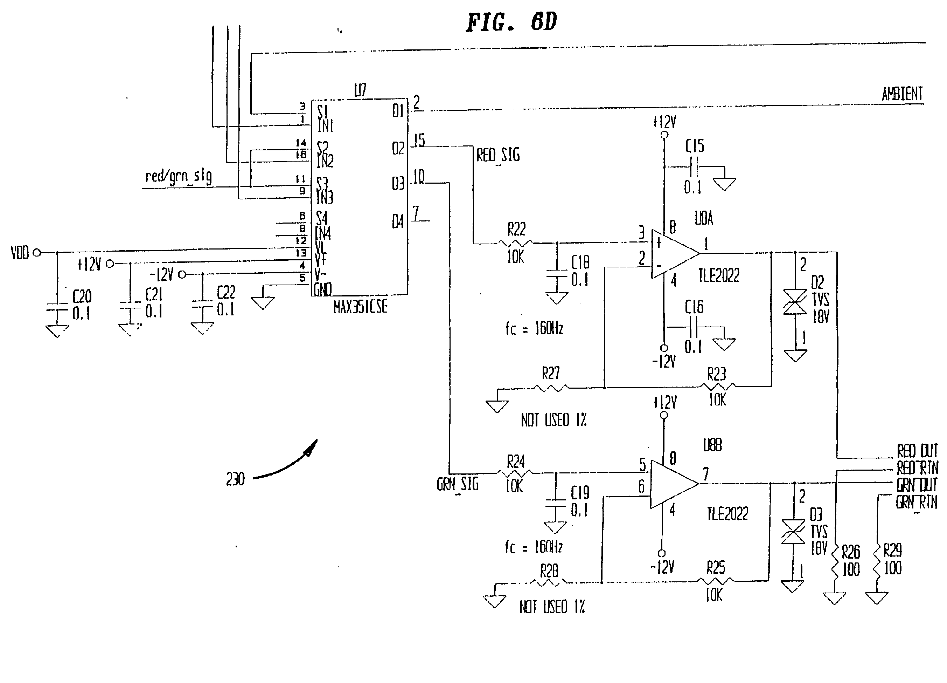

In general, optical sensor 58 is constructed and arranged to perform in-line characterization of fluids being transferred during the operation of cell processing system 10. Optical sensor 58 periodically samples fluids flowing through an optical chamber and provides data to control module 80. When optical sensor 58 detects a selected quality of the optically sampled fluid, it provides the corresponding data to control module 80, which, in turn, activates a selected valve within fluid distribution system 40. The activated valve redirects the flow of the fluid in accordance with the process.
A specific, currently preferred embodiment of optical sensor 58 according to the present invention is shown in Fig. 5. Referring to Fig. 5, optical sensor 200 includes a circuit board 202, a plastic mount 204, a source cover 206, a detector cover 208 and a soft gasket 210. A two-color light emitting diode 212 (shown in Fig. 6) is mounted on a source mount 214 and placed inside source cover 206. A silicon diode detector 216 (shown in Fig. 6) is mounted on a detector mount 218 and is located within the detector cover 208. Also, mounted within source cover 207 is a source aperture 213 having a 1 mm size hole. Source aperture 213, located in front of LED 212, is aligned with a detector aperture 217 located in front of silicon diode detector 216.

The light emitting diode is constructed to emit light of about 560 nm and about 640 nm. Preferably, the LED is AND176RAG made by Purdy Electronics Corp., 720 Palomar Ave., Sunnyvale, CA. The silicon diode detector is OPT210 made by Burr-Brown Corp., 6730 S. Tucson Blvd., Tucson, AZ 85706. Located on circuit board 202 is electronics 225 shown in Fig. 6.

After each power-up, control module 80 calibrates optical sensor 200 by taking the transmission data either without the cuvette or with the cuvette empty and comparing this to calibration data stored in the memory. Furthermore, a local controller 230 calibrates source 212 or detector 216 each time when a new cassette is located in fluid distribution system 40.

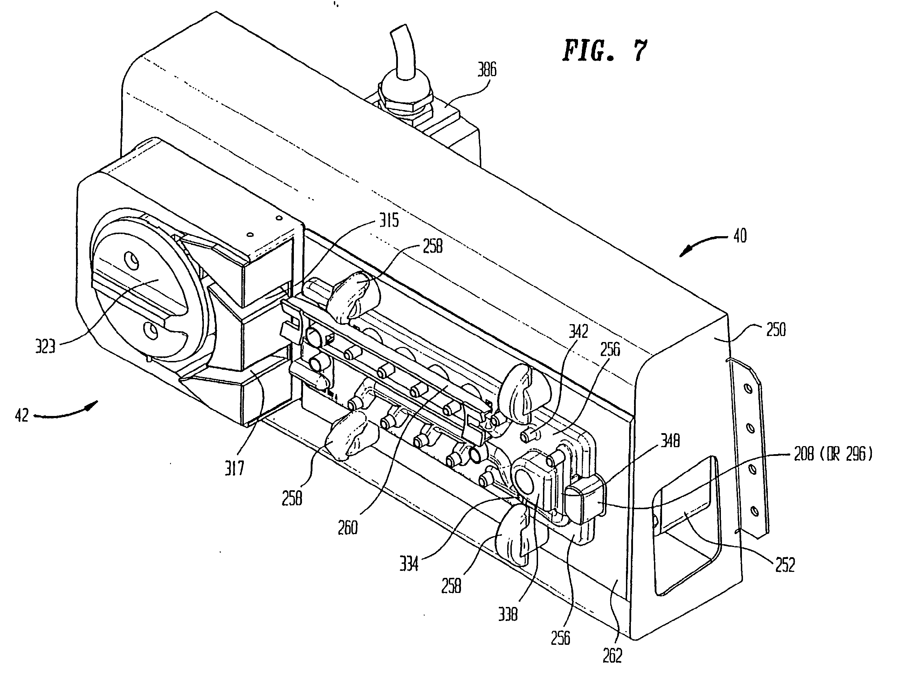

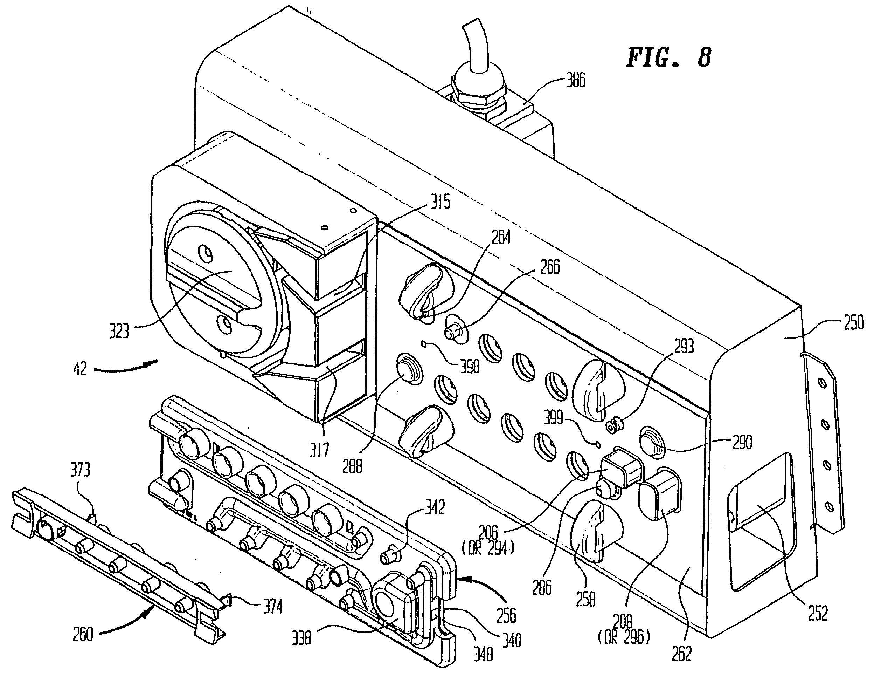

Figs. 7 and 8 show the arrangement of optical sensor 200 relative to fluid distribution module 40. The fluid distribution module is part of a fluid management system that coordinates the delivery of biological cells, process chemicals, solutions, fluids, reagents, etc. to conform with a processing algorithm executed by control module 80. Generally, the fluid distribution module controls the delivery of fluids from supply module 20 and cell module 12 to the processing module 60 (see Figs. 1 and 3), as well as the expression of fluids from the processing module 60. The fluid distribution module is a device comprised of pumps, valves, pressure management devices, and other components useful in the management of a multiplicity of fluids.

Fluid distribution module 40 is shown in Figs. 7-9. The fluid distribution module is part of a fluid management system that coordinates the delivery fluids including: biological cells, process chemicals, solutions, fluids, reagents, etc. to conform with a processing algorithm executed by control module 80. Generally, the fluid distribution module controls the delivery of fluids from supply module 20 and cell module 12 to the processing module 60 (see Figs. 1 and 3), as well as the expression of fluids from the processing module 60. The fluid distribution module is a device comprised of pumps, valves, pressure management devices, and other components useful in the management of a multiplicity of different fluids from different sources.

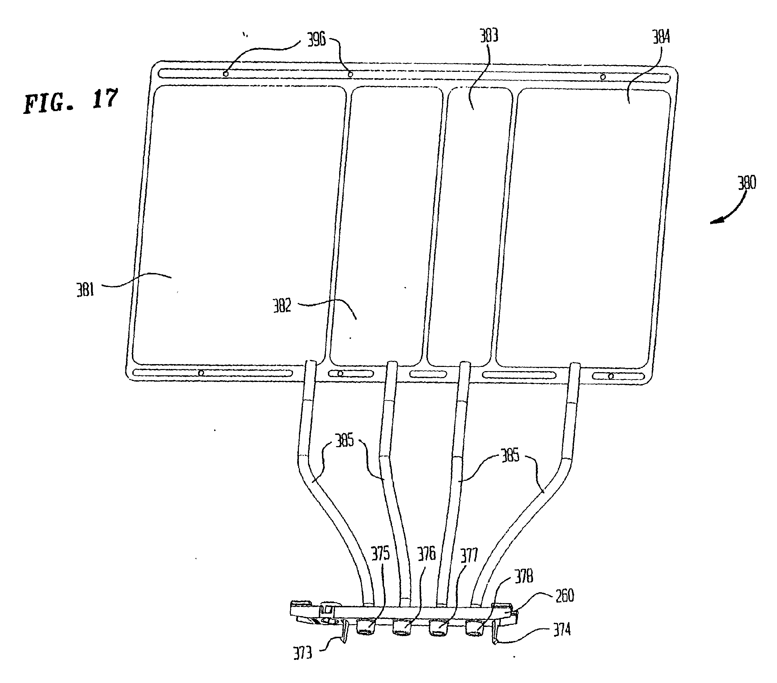

Referring to Figs. 7-9, the main components of the fluid distribution module are a housing 250, a pump valve assembly 252 mounted in the housing, and a distribution manifold 256 mounted on the housing on platen 262. The housing 252 can be formed from sheet metal. Also mounted on the housing 250 is peristaltic ("roller") pump 42. A connector 260 is attachable to the distribution manifold and receives tubing (see Fig. 16) from different sources of fluids to be transferred to the manifold.

The distribution manifold 256 includes a plurality of ports connected to interior runner channels for transferring fluid from one port to another. The ports are connectable to different sources or destinations of fluid.

The distribution module 40 is arranged so that the distribution manifold 256 is easily attachable to the housing 252 so that it may be a single use disposable device which can be replaced after the processing cycle is complete for a bag 16 of biological cells. The distribution manifold 256 is easily attachable and detachable to the housing through the use of spring knobs 258 (see Fig. 8). To attach the manifold, the spring knobs are rotated horizontally, the manifold is placed on platen 262, the spring knobs are pulled out, rotated vertically and released to bias the manifold against the platen.

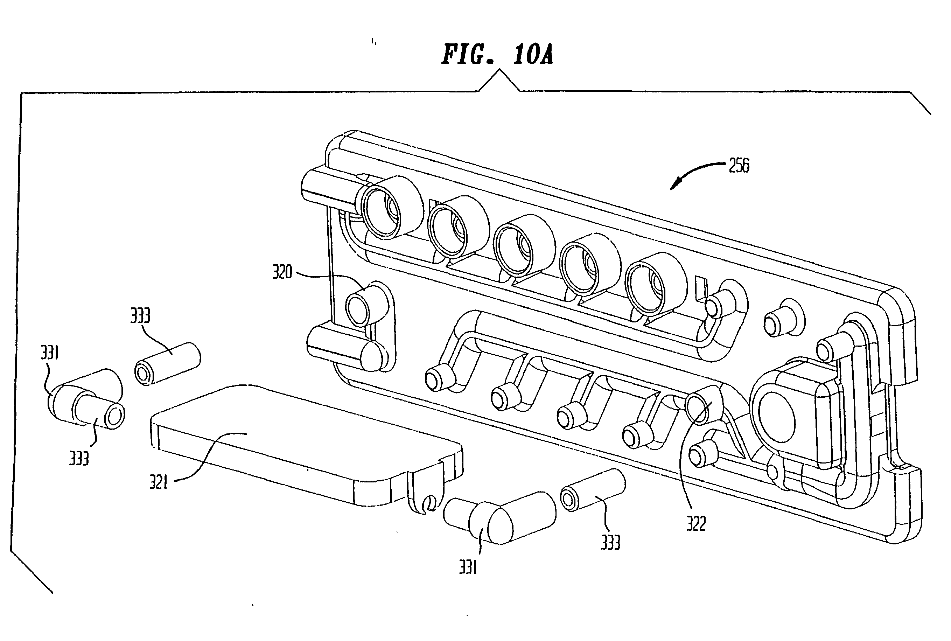

The platen 262 is seated in a recess 265 of housing 250. The platen 262 is an intermediary between the distribution manifold 256 and the pump valve assembly 252. The pump valve assembly includes a series of solenoids which can be energized to retract normally extended plungers 264, 266, 268, 270, 272, 274, 276, 278, 280, 282, 284 and 286 and thereby open corresponding valves 43-48 and 49-54 (Fig. 3) associated with corresponding ports 302, 304, 306, 308, 310, 312, 324, 326, 328, 330, 332, 334 (Fig. 10) on the distribution manifold used to transfer fluids to and from the manifold 256. As explained in detail below, a plunger, when extended, deflects a flexible membrane within distribution manifold 256 to close a particular port so that fluid cannot enter or exit the particular port; when the solenoid associated with a plunger is energized the plunger is retracted to open the associated port, or channel, and permit fluid entry or exit.

Also supported by the pump valve assembly 252 are: load cells 288 and 290 which are used to sense the fluid pressure at two points within the distribution manifold 256; a sterile air hose and filter 293; and optical sensor 58 including an emitter 294 and a detector 296. Hall effect sensors 298 are used to detect the position of the plungers 264-286.

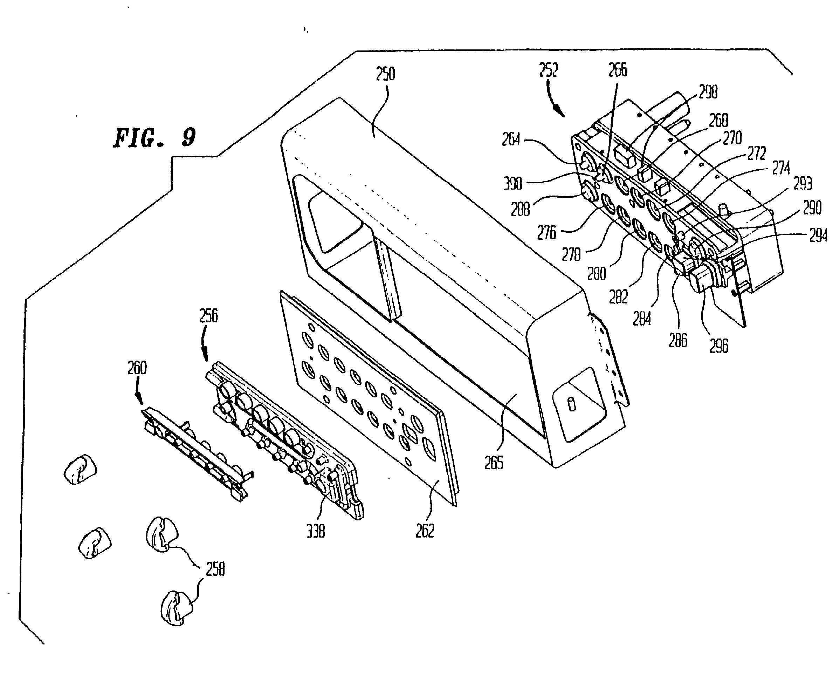

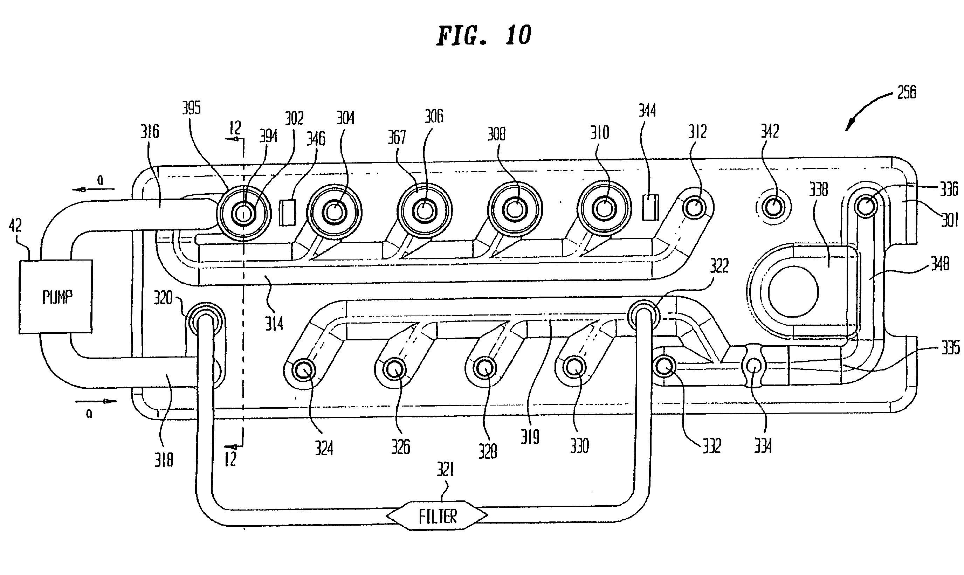

Platen 262 includes variously shaped holes 300 to accommodate the plungers 264-286, load cells 288 and 290, emitter 294 and detector 296 of the optical sensor, and sterile air hose 293 (see Figs. 8 and 9). To prevent fluids from entering the pump valve assembly 252, individual silicon plunger membranes can be placed over each plunger, as well as the two load cells, and will seal the respective holes 300 of the platen 262. Thus, the plungers 264 and 266 seen in Fig. 8 are covered by such membranes. In Fig. 8 plungers 264 and 266 are shown in the normal (i.e., non-energized) position in which the ports associated with the plungers 264-266 would be shut off. When attaching the distribution manifold to the platen 262 all of the solenoids are energized so the plungers do not interfere with the placement of the manifold.

The distribution manifold 256 is comprised of three main parts: a front plate 301, a flexible membrane 303 and back plate 305. The membrane is compressed between the front and back plate to form sealed channels in the manifold. The back plate is ultrasonically welded to the front plate, however, other methods of joining plastics may be used, for example, mechanical snaps, adhesives, solvents, etc. Like the platen 262, the back plate 305 also includes holes 307 which match with the holes of the platen 300 to accommodate the various elements of the pump valve assembly 252, and expose portions of the flexible membrane 303. For example, to close the valve associated with a particular port, a solenoid plunger passes through hole 307 of the back plate 305 and deflects the flexible membrane 303 toward the front plate to shut off fluid flow in a port or a channel of the front plate 301.

As seen in Fig. 10, ports 302, 304, 306, 308, 310 and 312, feed to a first distribution manifold channel 314. As stated above, these port are opened and closed by plungers 264, 266, 268, 270, 272 and 274. Different process chemicals can be fed via tubing to each port 302-312. For example, as described with reference to Figs. 3 and 4 above, enzymes A1/B and A2 can be attached to port 302 (from bags 21 and 22), DPP to port 304 (from bag 23), PEG to port 306 (from bag 24), storage solution to port 308 (from bag 25), PCI to port 310 (from bag 26), and saline solution to port 312 (from bag 74). Ports 302-310 are adapted to receive a connector 260 (described below) to which tubes from supply module 20 are attached.

Fluid will flow from a source connected to any of these ports into channel 314 (if the plunger for that port is retracted) and exit at outlet 316 when pump 42 is operating. Tubing connects outlet 316 to inlet 318. As shown schematically by arrows "a" in Fig. 8, the movement of fluid is from manifold channel 314. Fluid is transferred from 316 to 318 by a peristaltic pump (see Fig. 7) through which the tubing passes that connects outlet 316 to inlet 318. The pump has inlets 315 and 317 which receive the tubing, and a rotating roller 323 that rotates counterclockwise and continually pinches the tube along its length to generate a vacuum effect, sucking fluid from outlet 316 to inlet 318. Motor 386 causes roller 323 to rotate.

The fluid will proceed to port 320 and exit the manifold via tubing which is connected to a filter 321. The filter is a bacteriostatic filter having, for example, a.2 micron pore size manufactured by Pall Inc., and filters out contaminants which may be in the fluid. Two filters can be used in parallel to increase the rate of fluid flow which is slowed by the filter. The output of the filter 321 is coupled via tubing to another port 322 where the fluid enters a second manifold channel 319 of the manifold. Ports 320 and 322 do not have solenoid plungers associated therewith and thus are not valved ports. The filter is connected to ports 320 and 322 using elbow connccters 331 and small pieces of plastic tubing 333.

Ports 324, 326, 328, 330, 332 and 334 are also connected to manifold channel 319. Flow to these ports is controlled by solenoid plungers 276-286, respectively, corresponding to valves 49-54 (Fig. 3). These ports (and port 312) are adapted to be connected directly to tubing, unlike ports 302-310 which are adapted to receive connector 260 for reasons stated below. Ports 324-332 are two-way ports in that fluid can enter or exit from these ports. In the processing methodology described above with reference to Fig. 3, the ports are connected as follows: port 324 is connected to product bag 76, port 326 is connected to the processing module to provide a rinse saline solution, port 328 is coupled to the waste bag 72, port 330 is coupled to cell bag 16 (bypassing leukocyte filter 18), and port 332 is coupled to cell module 12 to receive unprocessed biological cells.

Using the distribution manifold 256, any fluid received on ports 302-312 can be distributed out any of ports 324, 326, 328, 330, 332 and 336. To distribute a fluid from ports 302-312 to ports 324, 326, 328, 330 or 332, gate valve 334 is closed by plunger 286 and the solenoid plunger associated with the desired port 324, 326, 328, 330 or 332 is energized and retracted to open the port so that fluid may pass. For example, saline solution received on port 312 can be pumped out of port 330 to dilute biological cells contained in cell bag 16 (bypassing leukocyte filter 18), or can be pumped out of port 326 to rinse the processing module 60. The rinse from port 326 is sent into and expressed out of the processing module and pushes the remaining cells in the line through port 336, valve 334 and out of port 324 to the product bag.

Alternatively, ports 324-332 could be kept closed, gate valve 334 opened, and fluid from any of ports 302-312 could exit out of port 336 to processing module 60. During cell processing described above, each fluid source connected to ports 302-312 is pumped into the processing module 60 at different times during the processing procedure (see Fig. 4).

A source of fluid received on one of ports 324-332 also could pass through gate valve 334 to a third manifold channel 335 and exit port 336 to processing module 60. For example, biological cells received from cell bag 16 connected to port 332 will travel through the manifold 335 and out of port 336 to the processing module 60. As with fluids received via any of ports 324-332, the cells travel from the bag 16 through the manifold 256 and to the processing module 60 via gravity, since the cell bag is placed above the distribution manifold and the distribution manifold is above the processing module 60.

Fluids can also be expressed off from the centrifuge 62 of the processing module 60. traveling into port 336, through channel 335 and gate valve 334, to any of ports 324-332. For example, the centrifuge 62 will express off waste and product to ports 328 and 324, respectively.

Third manifold channel 335 includes a cuvette 348 that leads to port 336 which is connected via tubing directly to processing module 60. The cuvette 348 is where processed fluid from processing module is detected by the optical sensor 58. The emitter of the optical detector is 294 is received in a cover 338 of the front plate 301 on one side of the cuvette, while the detector 296 is disposed in recess 340 on an opposing side of the cuvette. Thus, the detector can detect infrared light emitted through the fluid within the cuvette and detect the change to red blood cells that occurs after waste is expressed off by the centrifuge of the processing module 60. When the change is detected port 328 connected to the waste bag 72 is closed, and the process can either send the red blood cells back to the processing module 60 for further processing, or, if the process is finished, send them to the product bag by opening port 324.

The load cell 288 of the pump valve assembly 252 (see Fig. 10) is disposed beneath inlet 318 and port 320 to sense the fluid pressure being received in inlet 318. Load cell 288 senses high-pressure conditions which occur, for example, when the processing module is filled with fluid. For example, if fluid from one of ports 302-312 is being pumped to the processing module 60, when the processing module is filled the pressure will rise dramatically and be sensed by the load cell. The increased pressure signal is sent back to the control module which turns off pump 42. Sensor 288 also senses alarm conditions which can occur if there is a clog downstream from inlet 318 and port 320. A second load sensor 290 is placed beneath port 336 and senses the pressure in the centrifuge seal of the processing module 60. Thus, if the pressure in the seal at the centrifuge is too great, the processing can be discontinued or centrifuge speed reduced.

The remaining port 342 receives a sterile air hose 293 and filter from pump valve assembly 252 and is connected from front plate 301 via a tubing to processing module 60 which uses the sterile air to create a pressurized sterile environment. Openings 346 and 344 receive attachment fingers 373 and 374 (see Fig. 16) of connector 260. It should be noted that the particular arrangement of the ports, recesses, and manifold channels of the distribution manifold may be configured in numerous different ways to accomplish transfer of different fluids to different locations, and the invention is not limited to the particular arrangement shown in the figures.

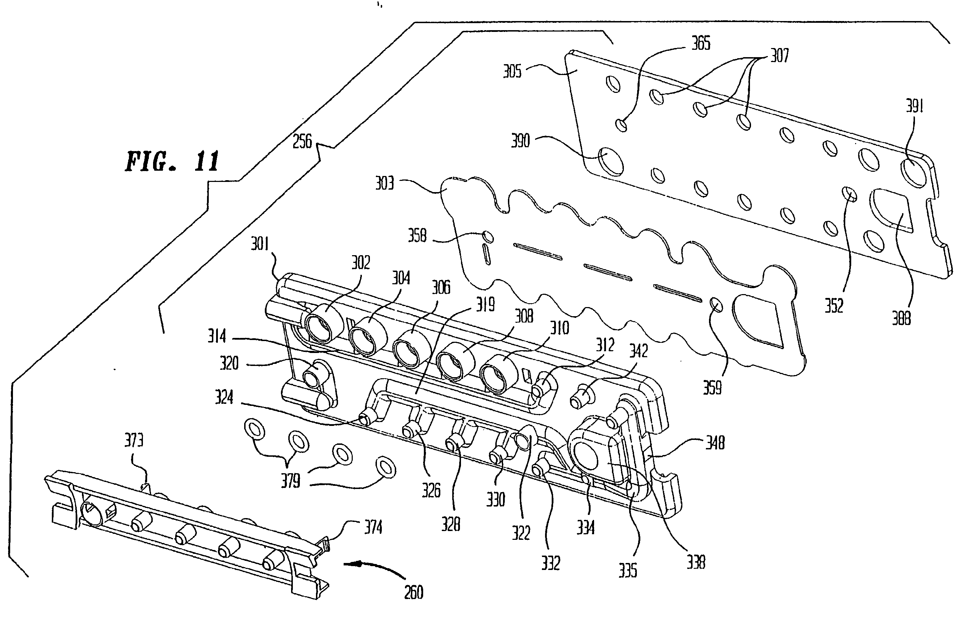

Referring to Figs. 11-13, the distribution manifold has three main components, a front plate 301, a flexible membrane 303 and a back plate 305. The front plate and back plate are injection molded plastic components made of amorphous clear polymer with high flexural modulus and good impact strength such as acrylic. Other materials may be used, for example, polycarbonate (PC), styrene acrylonitrile (SAN), polyester and copolyester, clear acrylonitrile butadiene styrene (ABS), polystyrene, polymethylpentene (TPX).

The flexible membrane 303 is made of a soft silicon material chosen for its ability to resist compression set and its load tensile modulus. Other materials can be used to form the membrane, such as thermoplastic elastomers (TPE). The distribution manifold 256 is assembled by sandwiching the membrane 303 between the front and back plate 301 and 305 and ultrasonic welding the front and back plate to one another. The front and back plate exert compressive force on the membrane.

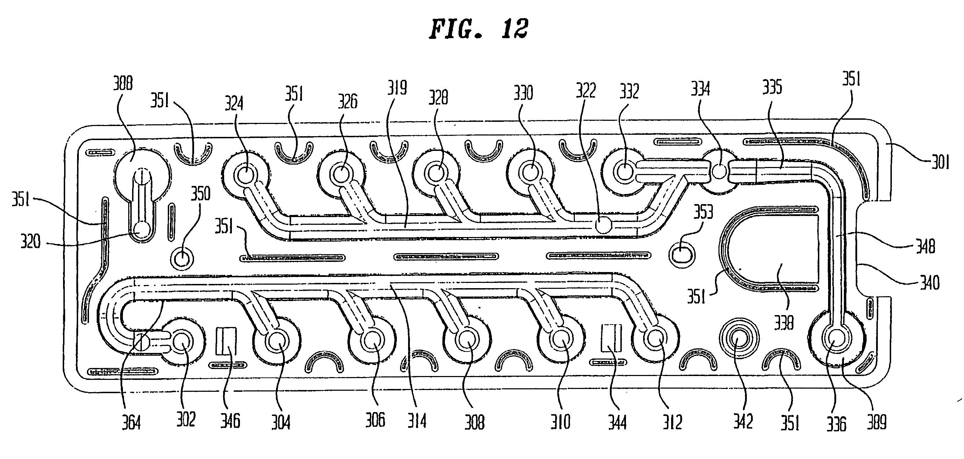

Fig. 12 shows a rear view of the front plate 301. Membrane 303 covers and seals with front plate 301 to form manifold channels 319, 314 and 335. The membrane 303 is compressed by the back plate 305 in order to form a good seal with the front plate 301 to prevent any fluid leakage out of manifold channels 314, 319 and 335, or any of the ports.

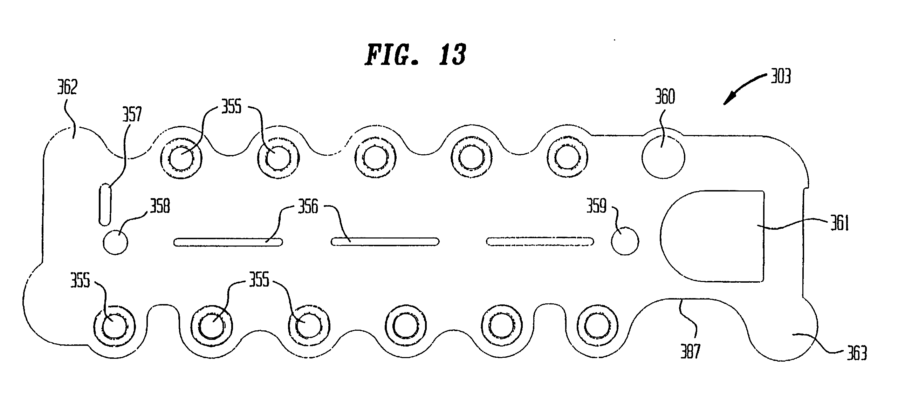

The distribution manifold 256 is constructed by laying the membrane 303 (as it is oriented in Fig. 13) over the rear of the front plate 301 (as it is oriented in Fig. 12). The side of the membrane that contacts the front plate is flat while the opposing side that contacts the back plate 305 includes bumps 355 which the solenoid plungers 264-284 are adapted to deflect to close the ports associated with and covered by the bumps 355 on the membrane. As seen by comparing Figs. 11 and 12, bumps 355 cover ports 302-312 as well as ports 324-332. A section 360 of the membrane formed without a bump and is used to close off gate valve 334 and receives a solenoid plunger (286) which is shaped slightly different to close off the gate valve 334 that connects manifold channels 319 and 335. The back plate 305 and the membrane 303 include apertures 388 and 361 through which the optical emitter passes.

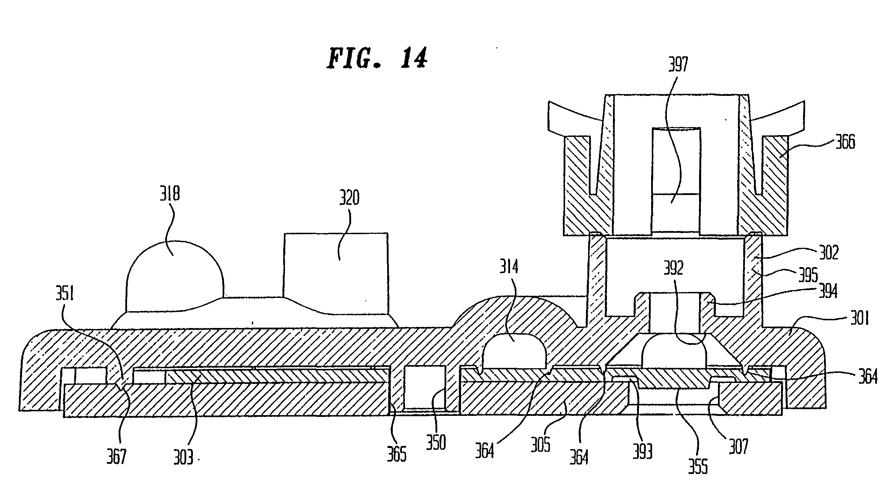

The rear surface of front plate 301 also includes a plurality of welding ribs 351 where the back plate 305 is to be ultrasonically welded to the front plate. The membrane is shaped to not interfere with the welding ribs and includes holes 356 and 357 which accommodate the ribs so they can be welded to the back plate. The weld is shown in Fig. 14 in which rib 351 is welded to back plate 305. The weld is formed at rib joint 367 in which part of the rib 351 is melted into back plate 305.

Areas 362 and 363 of the membrane 303 overlie areas 388 and 389 of the rear of the front plate. Load cells 288 and 290 contact the membrane at 362 and 363 through back plate holes 390 and 391, respectively, to sense the fluid pressure from fluid passing into inlet 318 and fluid passing into or out of port 336.

The front plate also includes pins 350 and 353 adapted to extend through the membrane 303 (through holes 358 and 359) and back plate 305 (through holes 365 and 352) in order to center the membrane 303 and back plate 305 properly on the front plate 301. The pins are hollowed (see Figs. 11 and 13) to receive mounting pins 398 and 399 of pump valve assembly 252 which extend through the platen 262. Pins 350 and 353 are slotted to accommodate for manufacturing tolerances. The slotting of pin 353 (see Fig. 12) is oblong to accommodate the greater horizontal tolerances due to the shape of the manifold 256.

The front plate further includes openings 346 and 344 for receiving attachment fingers 373 and 374 of connector 260. To properly position and hold the membrane in place as well as to form a seal, the front plate includes raised ridges 364 (see Fig. 14) which sink into membrane 303 when it is compressed between front plate 301 and back plate 305. Solenoid plungers are received in holes 307 in the back plate and will depress and deflect the exposed membrane at bump 355 to close a respective port. The plunger closes off the port by deflecting the membrane up to seal with surface 392 (see Fig. 14) of the front plate port. The membrane is slightly thinned surrounding the button 355 at 393 in order to assist the membrane in deforming to close the port.

The cross-section shown in Fig. 12 also shows a connector port 366 attached which is part of the connector 260 (see Fig. 7) which is attached to the face of the front plate. As seen in Fig. 8, ports 302, 304, 306, 308 and 310 are shaped to accommodate connector 260 rather than directly receive tubing as ports 324, 326, 328, 330, 332 and 336 do. Alternatively, the ports 302-310 can be formed like ports 324-336 to directly receive tubing if it is not desired to use connector 260.

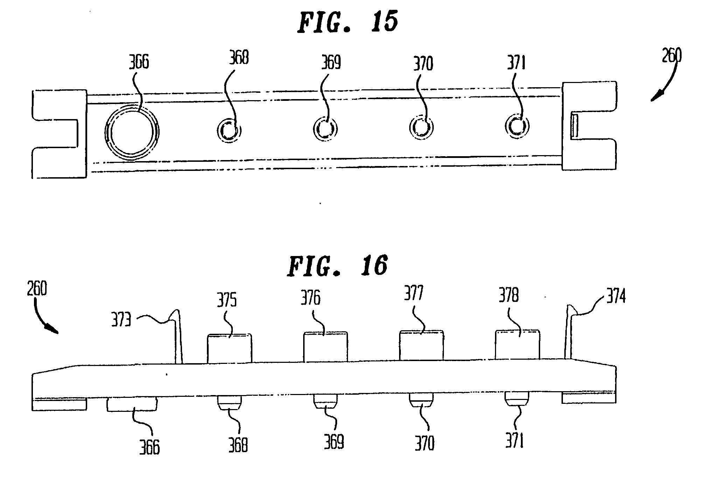

As seen in Figs. 15 and 16, the connector 260, which is made of injection molded plastic, includes cylindrical extensions 375-378 which are adapted to sit inside and to mate with an interior surface of ports 304, 306, 308 and 310, respectively. The connector assures that the process fluids from different sources are connected to the proper port of the distribution manifold. The cylindrical extensions are constructed to sit in between an inner ring 394 and an outer ring 395 of the port (see Figs. 10 and 14). O-rings 379 (see Fig. 11) are adapted to sit between the extensions 375-378 and ports 304-310 to provide a seal. Attachment fingers 373 and 374 snap into the front plate openings 344 and 346.

Ports 368. 369. 370 and 371 of connector 260 feed to respective extensions 375-378, and are attachable to tubing which is connected to a multi-compartment bag 380 as shown in Fig. 17. The bag 380 contains compartments 381, 382, 383 and 384 which can contain different types of processing chemicals, such as DPP, PEG. storage solution (AS3), and PCI. respectively. The bag can be shipped with connector 260 attached as shown in Fig. 17. The connector 260 will assure that tubes 385 are connected in the proper order to ports 302, 304, 306, 308 and 310 of front plate 301. The bag 380 is constructed of Cryovac M312, which is resistive to chemicals having a high pH like, for example, DPP and PCl. The compartments are formed by heat sealing two sheets of Cryovac M312 together. Holes 396 are used to hang the bag.

Port 366 (see Fig. 14) sits on port 302, and is for receiving an additional connector associated with a bag that holds the enzyme for processing biological cells. The enzyme bag connector snaps into slots 397 of port 366 and seals with an O-ring in port 302 in a manner similar to cylindrical extensions 375-378.

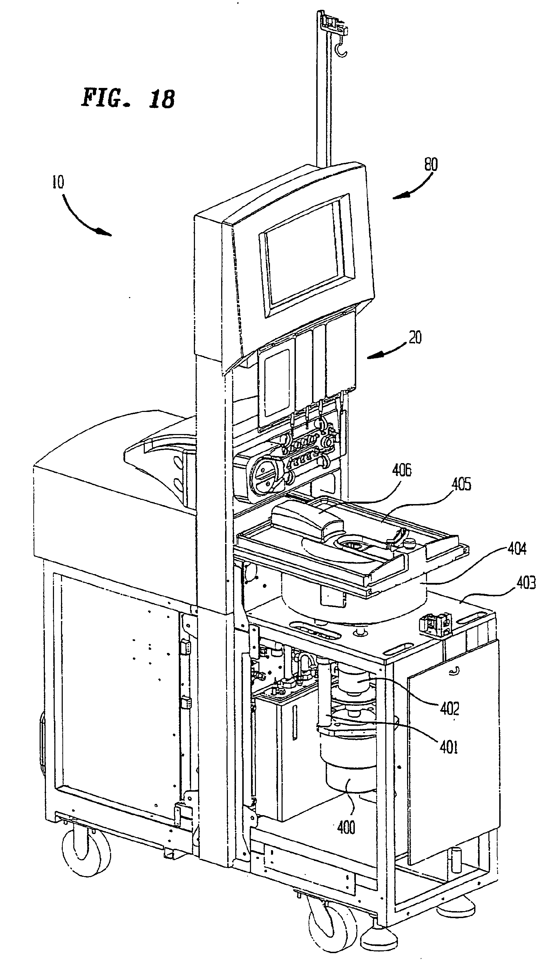

Fig. 18 depicts apparatus 10 in a left side perspective view showing the expressor system components more clearly in assembled and mounted relationship relative to the overall apparatus 10. In particular, a motor 400 for rotatably driving a chuck or rotor (described in detail below), separation posts 401, bearing housing 402, mounting plate 403, bucket 404, a sliding cover 405 and an infrared sensor housing assembly 406 arc shown in Fig. 18.

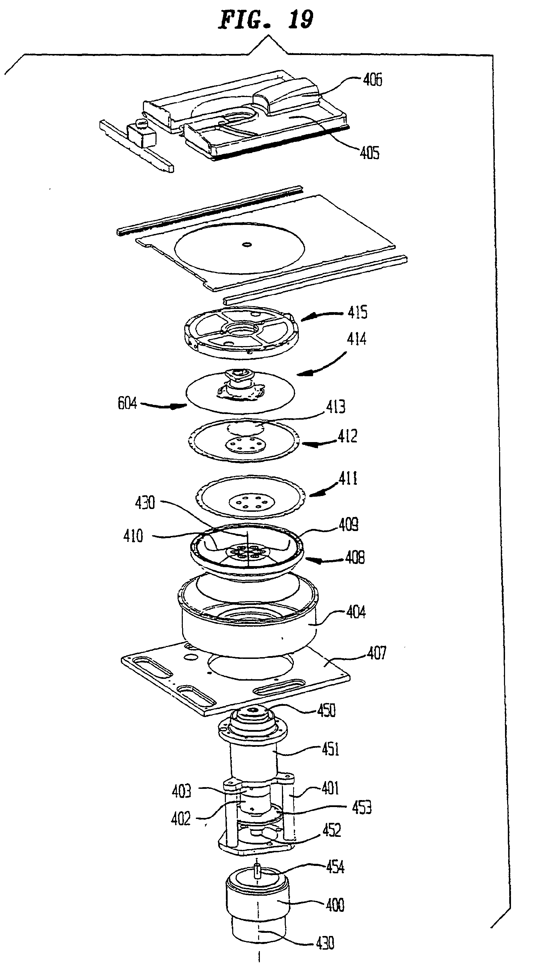

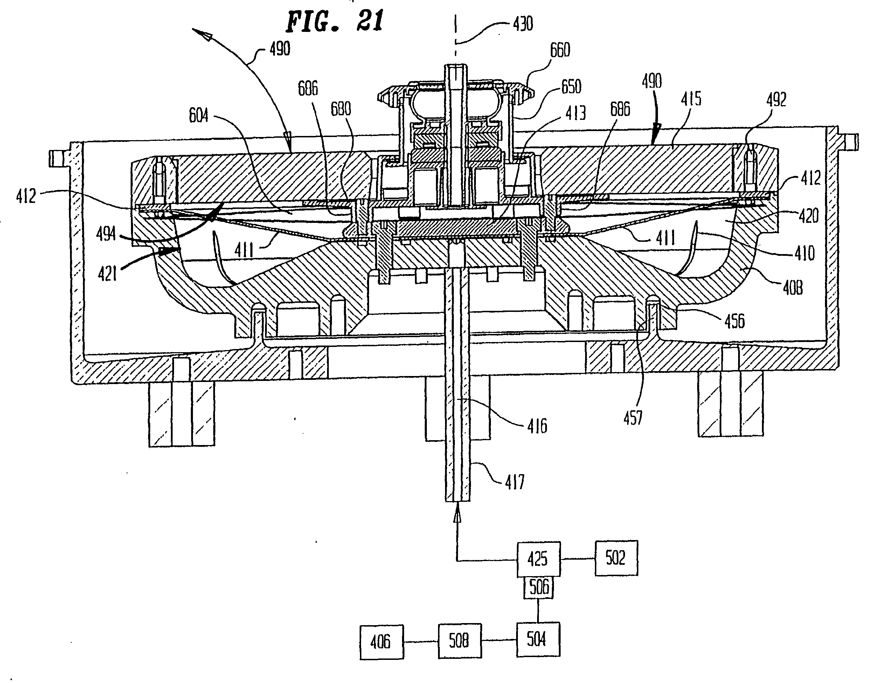

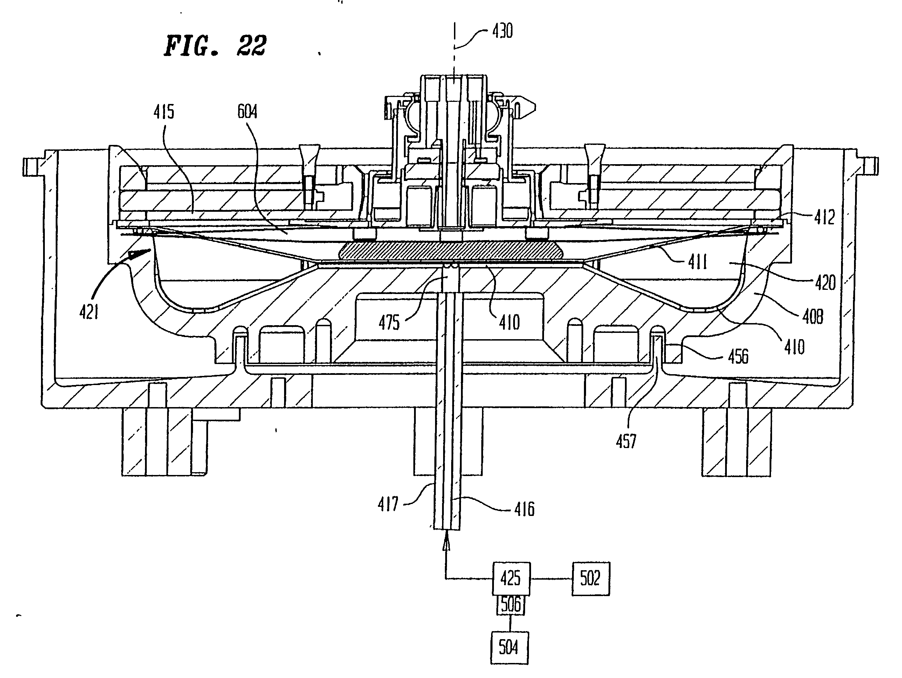

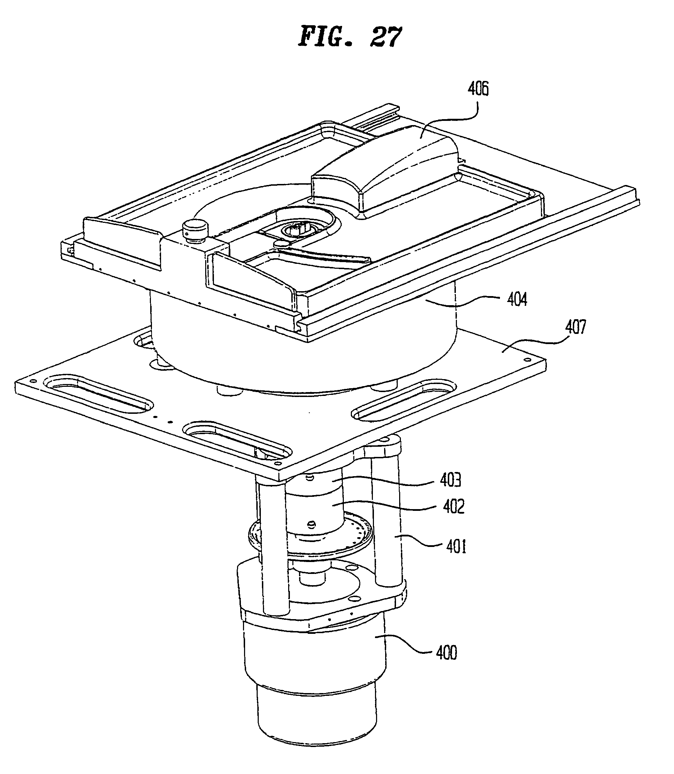

As shown in Figs. 19-27, the bucket 404 receives a chuck or rotor 408 which is rotatably drivable around central axis 430 via interconnection to motor 400 through shaft 450 which is housed within bearing housings 451-453 and coupling 452. As shown in Fig. 12, motor 400 rotatably drives shaft 455 which is connected to shaft 450 which is connected to chuck 408 which is mounted via grooves 456 and posts 457 (Figs. 21 and 22) within bucket 404 for rotation therein.

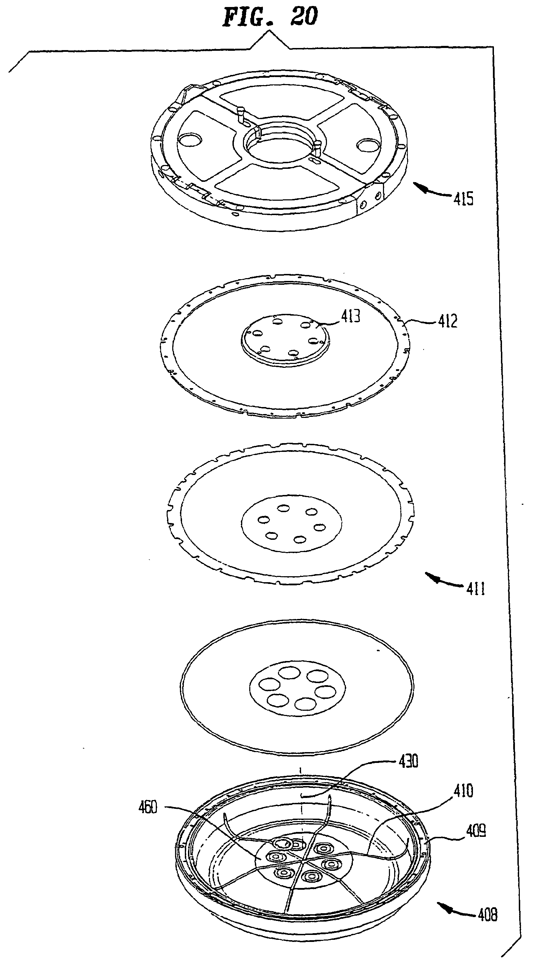

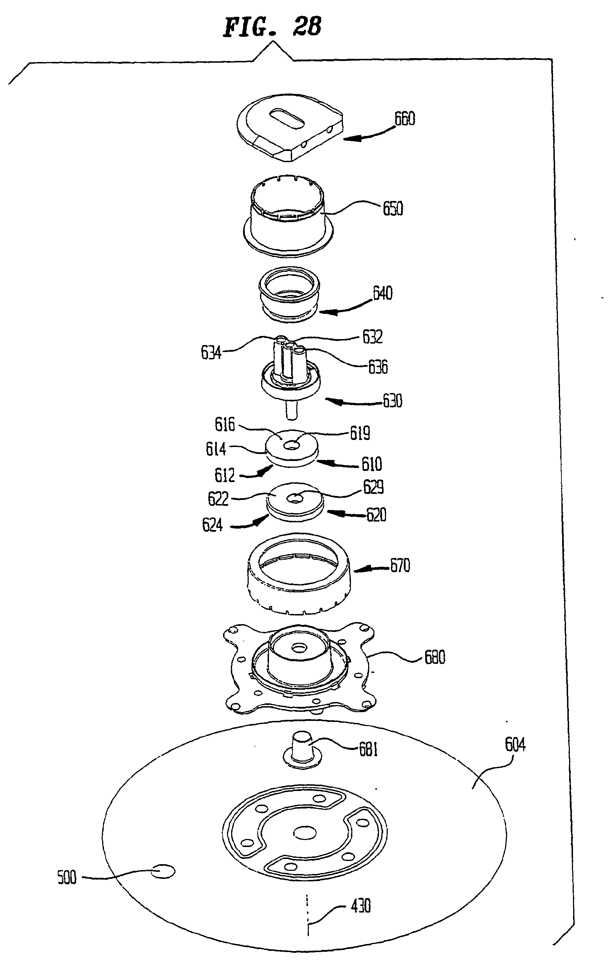

As best shown in Fig. 28, expressor fluid is pumped from an external source 425, i.e. external to the rotor, shaft and motor components, into a sealed annular space 458 which communicates with an axial fluid passage 416 through drive shafts 455 and 450. The axial fluid passage 416 communicates with a passage 459 in chuck 408 which communicates with grooves 410 on the inside surface of chuck 408 (Figs. 19 and 20). As best shown in Fig. 20, the fluid delivery grooves extend radially outwardly along a central flat circular surface 460 and further radially outwardly along the curved inside surface of chuck 408.

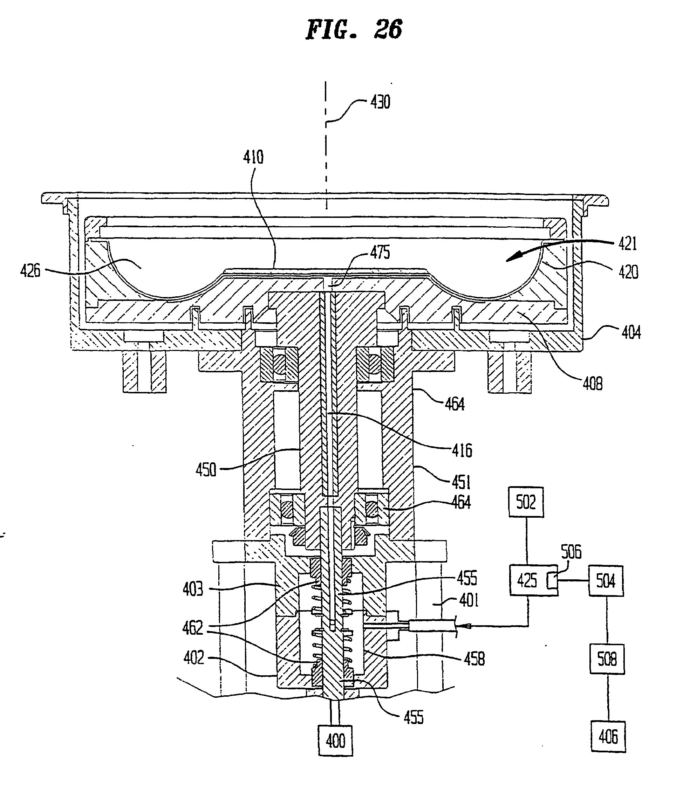

A pair of bearing seals 462, Fig. 26, enable the delivery of fluid from (and to) a stationary source 425 into space 458 and through the axis passage 416 of rotating shafts 455 and 450. Bearings 464, Fig. 26, rotatably mount shaft 450 within housing 451.

The chuck 408 has a round, donut or dish shaped chamber 421 (Figs. 21, 22, 23, and 24) within which the separation process occurs. The overall chamber 421 is divided into two separate enclosures, one being the space below flexible membrane 411, the other being the space within chamber 421 above membrane 411. The space below membrane 411 is sealably enclosed via the sealed mating of the underside of the outside circumference of membrane with the circumferential rim 409 (Figs. 19 and 20) of chuck 408 which is accomplished via the bolting of ring 412 (Figs. 21 and 22) to rim 409 with membrane 411 sandwiched therebetween. Membrane 411 is also sealably mated to the central flat surface 460 of chuck 408 via the bolting of chuck plate 413 (Figs. 20 and 21) to the center of chuck 408 with the center of membrane 411 sandwiched therebetween (Figs. 20 and 21).

The flexible membrane 411 comprises a resilient stretchable or flexible material typically an elastomeric material such as silicone, urethane or other suitable engineering elastomer such as Eastman Ecdel or DuPont Hytrel. The membrane 411 is non-permeable to fluid or gas and inert and/or non-reactive and/or non-porous to conventional aqueous and organic fluids and biological cells such as blood cells. The membrane 411 material is selected to be a material which stretches and contracts, is resilient, robust, and does not crease or deform upon stretching or contracting.

In the chamber space 426 above the top surface of membrane 411 within chamber 421 is mounted round fluid enclosure 604 (Figs. 19, 21 and 22) within which one or more fluid materials to be processed in some fashion is/are disposed. The fluid enclosure 604 comprises a flexible material, typically a sheet of plastic which is non-porous and inert to aqueous and biological fluids generally. The plastic material of the fluid enclosure 604 typically comprises polyvinyl chloride (PVC), polyethylene, inert multilayered coextruded plastics such as Cryovac M312. Eastman Ecdel elastomer or other equivalent flexible, inert plastic sheet material. The fluid enclosure 604 typically comprises an enclosure such as a bag (which may be disposable) or other donut shaped enclosure having at least one wall or side comprised of a sheet of the flexible plastic material, the outside surface of which faces the upper/outside surface of membrane 411.

The fluid enclosure 604 is typically filled with two or more fluids, such as an aqueous solution and a collection of biological cells which are to be separated from each other via centrifugal force or via gravity/sedimentation. For these purposes, a collection of cells which is capable of flowing relatively smoothly through conventional fluid flow tubing (e.g. having a diameter of at least about 0.10 inches) is considered to be a fluid or fluid material.

Where two or more fluid materials are input into or disposed in the fluid enclosure 604, each fluid material has a different density. The density of any and all materials which are input into or disposed within the fluid enclosure 604 is most preferably selected to be less than the density of the expressor fluid which is selected for input into the expressor space or chamber 420 (Figs. 21-25).

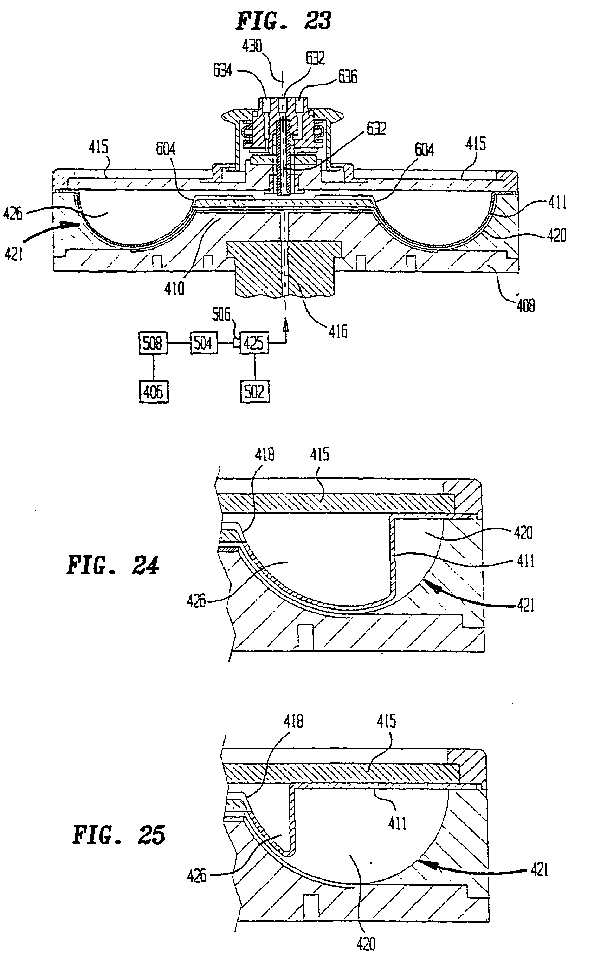

The density of the expressor fluid is preferably selected to be greater than the density of each of the materials disposed in the enclosure 604 so that upon rotation of chuck or rotor 408, the expressor fluid will preferentially travel to the outermost circumference of the chamber 421 under the centrifugal force, as best shown in Figs. 24 and 25, wherein in Fig. 24, a first selected volume of expressor fluid has been pumped into space/enclosure 420 and wherein in Fig. 25 a second greater volume of expressor fluid has been pumped into space/enclosure 420. Figs. 24 and 25 demonstrate that as the volume of expressor fluid is increased within enclosure 420, during the course of rotation of chuck or rotor 408, the flexible membrane 41 1 stretches/expands from the outermost circumferential edge of flexible enclosure 604 radially inwardly, thus compressing enclosure 604 radially inwardly and forcing the fluids to flow out of the enclosure 604, through exit port 470, sequentially according to the density of the fluid materials, least dense first to most dense last.

In a typical processing cycle, at the beginning, the membrane 411 is disposed in a position where the membrane 411 is held under suction pressure closely adjacent to the curved inner surface of the processing chamber 421, as shown in Fig. 13. A processing bag/enclosure 604 which, having a fill volume equivalent to space 426 (Fig. 13) is filled with a fluid containing biological cells disposed in an aqueous solution containing processing materials such as enzymes or buffers. The filled enclosure 604 is deposited in space 426 (Fig. 13) and the enclosure 604 is retained or fixedly held within space 426 via cover plates or doors 415 (Figs. 19-23), which are hingedly attached to chuck or rotor 408. At least the underside 472 of enclosure 604 (Fig. 19) comprises a flexible sheet material. The enclosure 604 is positioned within space 426 such that the flexible underside 472 of enclosure 604 faces and/or makes external surface to surface contact with membrane 411, as shown in Fig. 23. Expressor fluid is then controllably pumped from source 425 (Figs. 22 and 26) into axial channel 416 and flows upwardly to channel space 475 and then through grooves 410 into sealed space 420 (Fig. 22). During the course of pumping the expressor fluid into space 420, the chuck/rotor 408 is typically drivably rotated, the expressor fluid travels to the outermost circumferential volume of the sealed space 420 under centrifugal force (Fig. 24) and the membrane 411 is stretched/expands radially inwardly and continues to expand (as shown in Fig. 25) radially inwardly. As can be readily imagined as the volume of expressor fluid increases within space 420 (Figs. 24 and 25) the bag or enclosure 604 is compressed and the fluids contained within the bag/enclosure 604 are forced radially inwardly to flow out of an exit channel 632 or 636 which are sealably connected to and communicate with the interior space of enclosure 604.

In an alternative arrangement, relying on gravity force only, the rotor/chuck 408 may not necessarily be rotated during input/pumping in of the expressor fluid. In such case, the expressor fluid may fill the sealed expressor space 420 from the gravitational bottom of the chamber 421 and expand the space 420 from the bottom upwardly compressing the bag/enclosure 604 from the bottom upwardly. Because the two or more materials disposed within the bag/enclosure 604 have different densities, the two or more materials will separate from each other within the bag/enclosure 604 over a certain period of time (depending on the densities of the fluid materials) under the force of gravity. Once the materials have been allowed to separate over time, the expressor fluid may be pumped into space 420 and the gravitationally separated materials may be compressed out of an exit channel 632, 636 sequentially according to their densities, least dense first to most dense last.

The expressor fluid is preferably selected to have a lubricating effect on the rotating bearing seals 462 (Fig. 26) and selected to be non-corrosive and not overly viscous. Most preferably the expressor fluid is a mixture of glycerine and ethylene glycol in a ratio of between about 40:60 and about 60:40, most preferably about 50:50 (having a density of about 1.15) which, for the vast majority of biological fluid applications, has a density greater than the density of the biological fluids. Other examples of expressor fluids having a density greater than most biological fluids are glycerol and ethylene glycol diacetate which are less preferred. Any stable, non-corrosive, relatively non-viscous fluid preferably having a density greater than the density of each of the fluid materials disposed in the enclosure 604 may be used as an expressor fluid.

The enclosure 604 which receives the fluids to be processed is a sealed enclosure, preferably having a fluid input port 632, 636 which is/are readily sealably attachable to a readily selectable source of fluid, such as wash or preservative or compacting fluid or buffer or biological cell containing or enzyme containing fluid. Such selectable sources of input fluids may be each connected to a manifold or fluid management apparatus (e.g. a subassembly or subsystem of module 40, Fig. 1) which can be programmed or otherwise readily controlled to deliver a selected fluid for input to the enclosure 604. An output port of such a manifold or fluid management apparatus is readily sealably connectable to an input port 632, 636 of the enclosure 604.

As shown in Figs. 13, 28, 29 and 30, several fluid communication ports 632, 636 are provided, each port being both an input and an exit/output port.

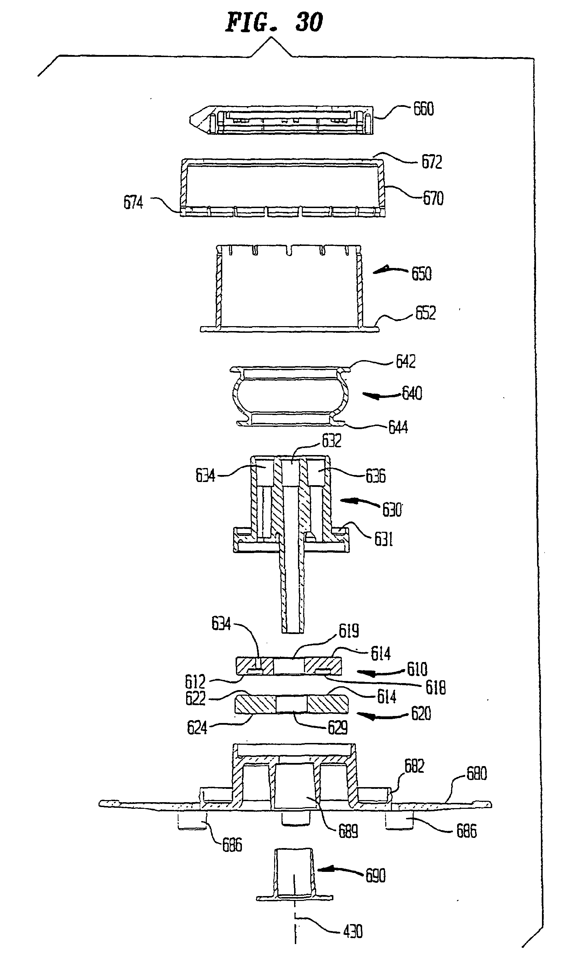

One fluid communication port 632 may be utilized for inputting and outputting a biological cell material and the other port 636 might be utilized for inputting/outputting a processing fluid (e.g. buffer or enzyme containing aqueous solution). The ports 632, 636 may be sealably connected to a fluid management apparatus as discussed with reference to Fig. 1, wherein a series of valves are utilized to separately enable flow into, out of or through one port or another at any given time. The input/output ports 632, 636 of the enclosure 604 sealably communicate with the interior of enclosure 604 via the assembly and fastening together of rotating seal components 630 (body), 610 (upper seal), 620 (lower seal), 670 (header clamp), 680 (base), 681 (plug) (Figs. 28-30) together with bag/enclosure 604 so as to provide several sealed fluid communication ports 632, 636 into and out of the interior 426 of the enclosure 604 (Fig. 13). Another channel 634 as shown is provided in the rotating seal components 630 and 610 (Figs. 28 and 30) for input of sterile gas between and around the undersurface 612 and upper surface 622 of seal components 610. 620 which mate and rotate with respect to each other.

Most preferably, when biological cells are input into enclosure 604 together with a selected processing fluid having a predetermined composition, the ratio of the amount of biological cells and processing fluid is maintained constant between any two or more processing cycles, i.e. the processing conditions to which any two separate aliquots of biological cells are subjected is identical as between separate processing cycles.

As can be readily imagined, the volume of fluid input into the processing enclosure 604 at the beginning of any particular processing cycle may be selectively varied, i.e., the processing enclosure 604 may be filled anywhere from 0-100% of its volume capacity, with the remaining unoccupied volume of the processing chamber 421 being selectively filled up by inputting or pumping in an appropriate amount of expressor fluid into enclosure 420. Most preferably, the maximum volume or capacity of the processing enclosure 604 is approximately equal to or slightly less than the volume of the chamber 421. As described above, the hinged doors 415 are pivotable 490 (Fig. 21) between open and closed positions, the doors 415 being shown in the closed position in Figs. 21-23. When the doors are opened, the bag enclosure 604 is insertable into chamber 421 and when the doors are closed as shown in Figs. 21-23, the bag/enclosure 604 is firmly held within the volume of chamber 421. The doors are lockable into the closed position shown in Figs. 21-23 by conventional means such as a via spring biased hinges 492 or other conventional means such as clasps, clamps or the like. The undersurface 494 of doors retains the bag/enclosure 604 within chamber 421 and provides a stationary surface against which the bag/enclosure 604 engages and thereby is forced to compress under the opposing pressure exerted by the flexible membrane 411 on the flexible wall of the bag/enclosure 604 when the space 420 is expanding as described for example above with reference to Figs. 24 and 25. Suitable alternative mechanisms to doors 415 may comprise, for example, a plate or disc which is slidable into a stationary position equivalent to the closed position of doors 415 (Figs. 21-23).

The apparatus includes a sensor for monitoring the temperature of the fluids disposed in the processing enclosure 604. The temperature sensor may comprise an infrared IR thermocouple 406 (Fig. 19), which detects IR radiation in a range of about 2 µm to 10 µm emitted through an IR transparent window disposed over the bag/enclosure 604. The transparent window typically comprises ZnSe and is coated by a 0.5 mill layer of parylene N. The parylene N coating is used to protect the transparent window although it has some absorption of the IR radiation. Other conventional temperature sensors may also be employed.

The IR thermocouple (e.g. IR t/c.03-J-80F/27C made by Exergen, Corp., 51 Water Street, Watertown, MA 02172) integrates the detected IR energy to determine the temperature of the fluids. This temperature is corrected for the local air temperature or ambient temperature between the transparent window and processing enclosure 604. This air temperature is measured by a second temperature sensor that is a Si diode temperature sensor. The data from the Si diode is used for correcting the IR data.

Most preferably, the temperature of the fluids disposed in the processing enclosure 604 is controlled by controlling the temperature of the expressor fluid which is input into the expressor chamber/space 420. Preferably, the source of expressor fluid 425 (Figs. 21-23 and 26) which is pumped via pump 502 into annular space 458 (Fig. 26) and through channel 416 and ultimately into chamber space 420, is connected to a fluid heating and/or cooling device 506 (Figs. 21-23 and 26), which is controlled by a heating and/or cooling controller 504. The expressor fluid is typically circulated through a reservoir, within which, the fluid is in thermal contact with certain devices that transfer thermal energy to or from the fluid in response to a control algorithm. These thermal device may include Peltier Devices, electric resistance submersion heaters, air cooled radiators, or other similar devices or some combination of these types of thermal transfer devices. The expressor fluid which travels through channel 416 and grooves 410 makes contact with the surfaces of rotor or chuck 408 and the membrane 4011 and the shafts 450, 455. Rotor 408 and shafts 450, 455 are typically comprised of a heat conductive material such as metal (e.g. steel, iron, copper, aluminum or the like) and are thus readily heated or cooled to the temperature of the expressor fluid with which they are in contact. The temperature of the expressor fluid is thus readily conducted to the fluids disposed in the processing bag/enclosure 604 via the rotor 408, shafts 450, 455 and through the flexible membrane 411 with which the flexible wall of the bag/enclosure 604 makes contact within chamber 421. Thus, by controlling the temperature of the external source 425 of expressor fluid, the temperature of the entire processing system including the interior chamber 421 may be controlled.

The temperature of the fluid being monitored by sensor 406 may be input to a program or circuit 508 connected to controller 504 (Figs. 18, 21-23, and 26). The program or circuit 508 preferably includes a subroutine for automatically directing the temperature controller to heat or cool the temperature of the expressor fluid source 425 to a predetermined constant temperature or series of temperatures over a predetermined period of time. The program 508 preferably includes a predetermined algorithm which uses the temperature information signal which is input from sensor 406 to direct control of the temperature controller 504 and heater and/or cooler element 506 such that the temperature of the external source 425 of expressor fluid is varied depending on the temperature signal input from sensor 406. The temperature of the source 425 may be cooled by simply terminating heating of the expressor fluid 425 thus allowing the fluid 425 to passively cool by self-radiation of heat rather than by proactive cooling.

Processing module 60 includes a "rotating seal" that is a seal created between moving and stationary components of the centrifugal element. The seal acts as a barrier between the interior portion of the system in which processing occurs, which is desirably maintained as microbe-free as possible, and a nonsterile environment which, at least during a portion of the operation of the system, is in communication with the environment external to the system. The rotating seal also prevents the dispersal of microbes (*e.g.* viruses) which may exist in a cell sample into the external environment.

The rotating seal comprises an upper element and a lower element, wherein one element rotates during at least a portion of the operation of the cell processing system. The rotating seal surrounds an axial opening through which cells and/or cell elements and processing materials are intended to pass during processing.

Referring again to Fig. 21, protruding from the top of the centrifuge bucket are the first port 632, the header shield top 660 and the header shield bottom 650. The rotating seal apparatus is mounted to the chuck by mounts 686 protruding from the base 680. The mounts mate with opposing projections fixed to the chuck, thereby transmitting the rotating force of the chuck to the lower parts of the rotating seal apparatus and the processing container to which the base 680 is mounted.

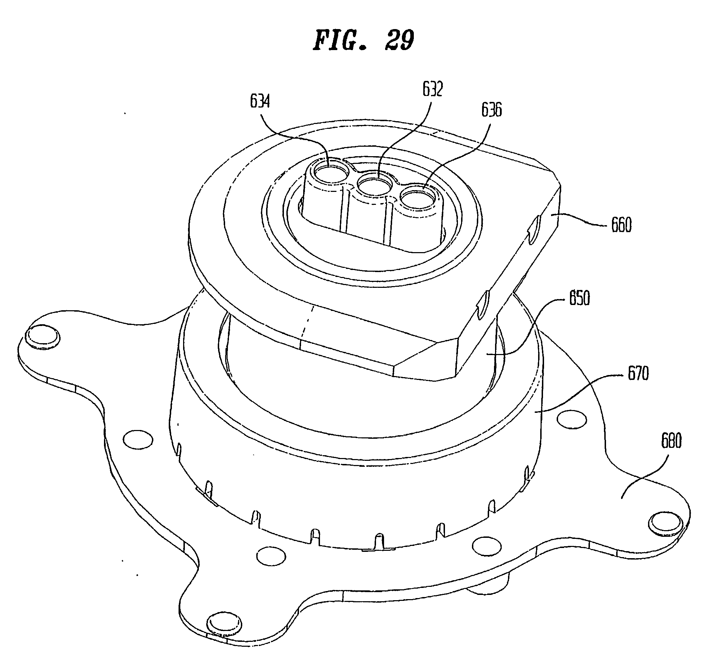

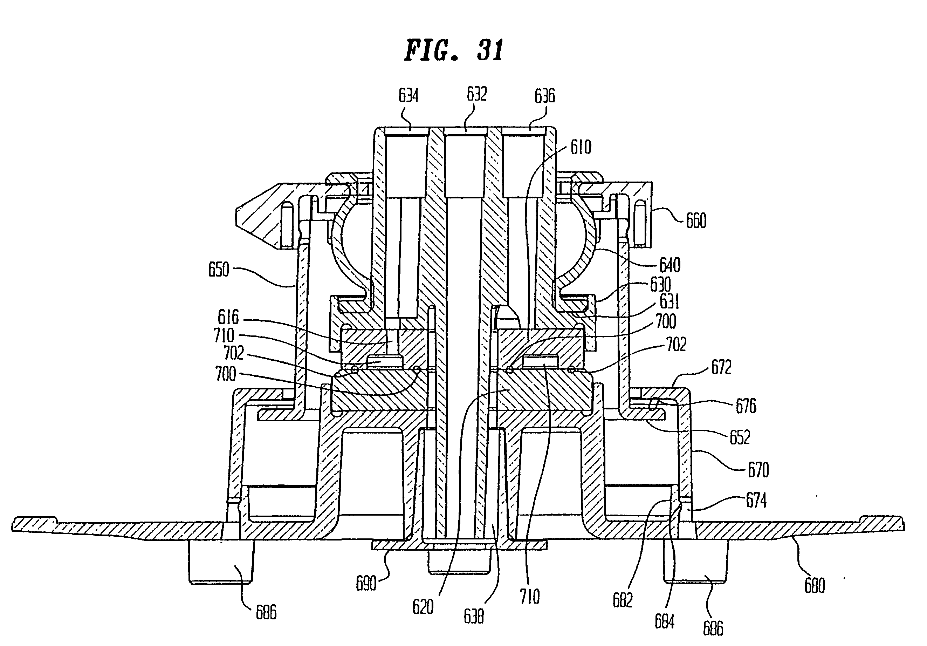

As described above, Fig. 29 depicts an assembled rotating seal apparatus. The header shield assembly is comprised of the shield top 660, the shield bottom 650 and the shield clamp 670. The shield clamp includes an inwardly directed flange 672 which overlaps an oppositely directed flange on the shield bottom. Alternatively, the shield clamp can have a smaller diameter than the shield bottom, and have an outwardly directed flange to overlap an inwardly directed flange of the shield bottom. The shield clamp is mounted on the base 680, which in turn is mounted on a processing container. As depicted in Fig. 31, the base 680 includes a flange 682 which includes a outwardly directed protrusion 684. When the shield clamp is mounted on the base, the protrusion 684 fits into the indentation 674 on the inside surface of the shield clamp, thereby holding the header shield assembly together and preloading the spring 640 to create contact between the sealing surfaces. Ports 632, 634 and 636 are included as inlets and outlets for materials passing through the rotating seal apparatus to a processing container and for providing materials to internal portions of the rotating seal apparatus. These arc described in greater detail below.

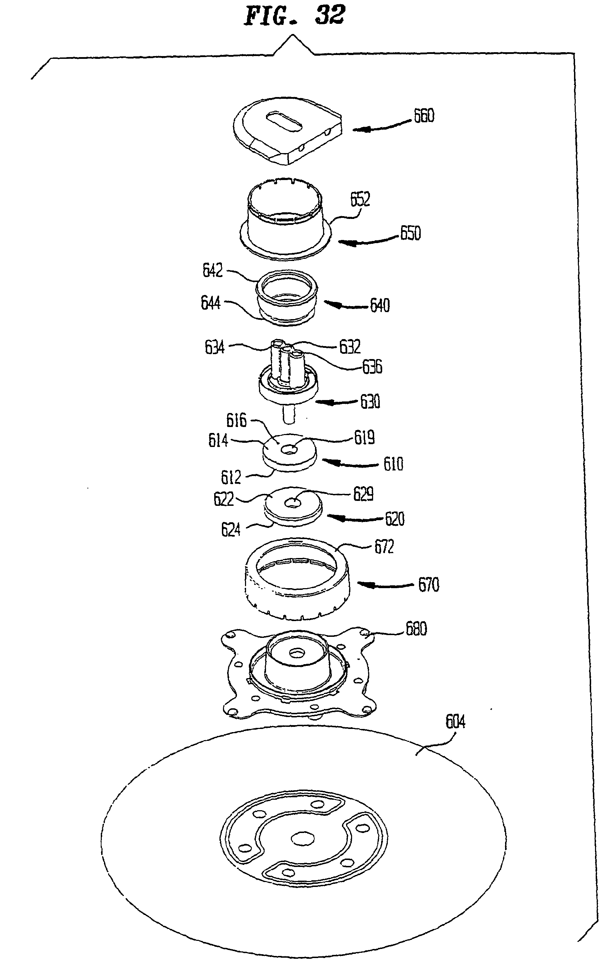

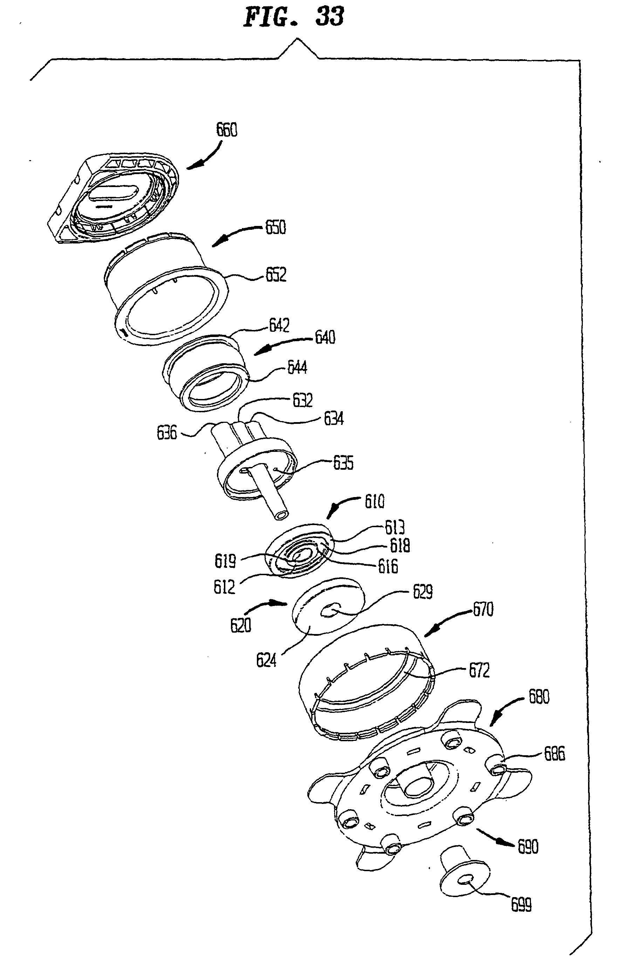

Referring to Figs. 30-33, the rotating seal comprises an upper sealing member 610 and a lower sealing member 620. As shown in Fig. 31. when biased together, the contact of the scaling members creates a plurality of annular seals. A first seal 700 is formed between sealing surfaces 612 and 622, and a second seal 702 is formed between sealing surfaces 613 and 622. As shown in Figs. 31, 32 and 34, the sealing face of the upper sealing member 610 is formed into two sealing surfaces 612 and 613 as lands surrounding a groove 618. The groove forms the upper boundaries of an annular space 710 between the concentric seals 700. 702. The groove can be cut from or molded into the upper sealing member as desired.

As depicted in Figs. 30-33, the sealing face of the lower sealing member 620 is not formed into lands and grooves: rather, the sealing surface 622 forms the plurality of concentric seals in combination with the sealing surfaces 612 and 613, and forms the annular space in combination with the groove 618. In alternative configurations of the rotating seal, the lower sealing member can include the topography of lands and grooves and the upper sealing member can be planar. In still other arrangements, both the upper and the lower sealing members can include lands and grooves. The sealing surfaces preferably are planar, although other geometries also can be used provided that a close fit can be achieved between stationary and rotating members of the rotating seal.

The upper and lower sealing members 610, 620 also each define axial openings 619 and 629, respectively. Upon assembly of the sealing members in axial alignment, the axial openings, the first seal, the annular space, and the second seal are positioned concentrically relative to each other.

The annular space 710 can be in communication with the external environment, and preferably is in gaseous communication through the channel 616. The channel can be formed in either of both of the sealing members 610, 620. Preferably, the annular space 710 constitutes a sterile chamber. Further seals, separated by additional annular spaces, may also be included in the rotating seal apparatus.

The rotating seal apparatus depicted in Figs. 29-33 also includes a body 630 having ports 632, 634 and 636 which serve as inlets and/or outlets for material passing into and out of a processing container to which the rotating seal apparatus is mounted. The first port 632 traverses the axial opening of the rotating seal apparatus, terminating at plug 690. The first port preferably serves as an inlet into the processing container for cells which are to be processed. The first port additionally serves as the outlet for processed cell following the execution of processing steps. The first port can be connected to a number of tubes, fluid handling manifolds, valves etc. as will be known to one of ordinary skill in the art.

The fluid port 636 is in fluid communication with the annular space 638 bounded by the exterior surface of the first port and the walls of the axial openings 619, 629, 689, 699 of the upper sealing member 610, lower sealing member 620, base 680 and plug 690. The fluid port 636 connects to the annular space below. The fluid port 636 and annular space 638 may be for passage of processing materials such as wash solutions, buffers, enzymes and the like into the processing container. The fluid port 636 and annular space 638 also are used for passage of waste materials out of the processing container. This outlet function also serves a temperature regulation function. As the sealing members of the rotating seal apparatus turn against each other, local frictional heating of the rotating seal apparatus above room temperature occurs. Passage of waste materials, which are at temperatures at or below room temperature, out of the processing container through the annular space 638 and fluid port 636 contact the first port 632 and thereby lower the temperature of the first port. The cooled first port does not heat cells as an uncooled port would upon passage of processed cells out of the processing container through the first port. As with the first port, the fluid port can be connected to a number of tubes, fluid handling manifolds, valves etc. as will be known to one of ordinary skill in the art.

The gas port 634 is in gaseous communication with the annular space 710 between the concentrically spaced seals 700, 702. The gas port preferably serves as the inlet for providing sterile air (or other gas) to pressurize the annular space 710. The gas port can be connected to a number of tubes, filters, valves etc. for providing a sterile supply of gas as will be known to one of ordinary skill in the art.

The.base 680 and plug 690 fit together as depicted in Fig. 31. A single unitary base/plug combination also could be used as desired. The base 680 serves both as a mount for the lower seal member 620 and as a mount for the shield clamp 670 by means of the flange 682 and protrusion 684. The base is mounted on the processing container to provide fluid communication of the first port 632 and the fluid port 636 with the interior of the processing container. A plurality of mounts 686 can pass through sealed portions of the processing container can be mounted on the chuck of a centrifuge to communicate rotation of the centrifuge chuck to the base, processing container and lower seal member of the rotating seal apparatus. Other means of securing the rotating seal apparatus and processing container to the centrifuge for providing rotation to the processing container are well known to one of ordinary skill in the art.

The spring 640 is depicted in Figs. 30-33, and comprises a hollow generally cylindrical-shaped elastic member having bowed sides. The spring is disposed between the header shield top 660 and the body 630. As depicted, the spring is provided with flanges 642, 644 at its upper and lower ends. The lower flange 644 fits into an annular recess 631 formed in the body. The top flange 642 fits against the header shield top. Upon assembly of the rotating seal apparatus by snapping the header shield clamp against the base, the spring is deflected from a first position to a second position which provides a "pre-load'' of contact forces to the seal members. When the rotating seal apparatus is enclosed in a centrifuge, the spring can be deflected to a third position of greater deflection which provides an increased contact force to the seal members, thereby creating an enhanced seal.

In contrast to helical springs, the cylindrical spring provides a constant biasing force over a wide range of compression. The spring has a height *h* which describes the axis of deflection or compression, a width w which describes the diameter of the spring in nondeflected or deflected positions, an thickness *t* and an arc *a*. The height, when the spring compressed from the first position, is reduced from *h*₁ to *h*₂. Similarly, the spring width is increased on compression from *w*₁ to *w*₂. The spring provides constant biasing force over a Δ*h* of at least 10%, preferably at least 20%, more preferably at least 30% and most preferably at least 50%. Further, the spring provides constant biasing force over a Δ*w* of at least 1%, preferably at least 2%, more preferably at least 5% and most preferably at least 10%. The range of compression over which the biasing force is constant also can be described by the ratio of Δ*h*:Δw, wherein the spring experiences a relatively large Δ*w*, for a corresponding Δ*h*. The thickness *t* of the spring should not be too great as to hinder the bowing of the cylinder sides on compression of the spring. A range of thicknesses and arcs will be useful to provide proper bowing; these ranges can easily be determined by one of ordinary skill in the art with no more than routine experimentation, and may depend on the particular elastomeric material chosen for manufacture of the spring. The appropriate thickness and arcs can be expressed in terms of ratios of *h:t* and *h:a,* when *h, t,* and *a* are measured in deflected or nondeflected positions.

The rotating seal apparatus may be validated as "closed" devices, and thereby produce product with longer shelf life than prior art rotating seal devices. A second annular seal provided circumferentially around the first or inner seal provides further assurance of seal integrity. To still further promote the sterility of the interior of the seal and processing container, the annular space 710 can be filled with sterile air and, further, a pressure differential may be created such that the sterile air in the annular space is at a pressure higher than the pressure in the surrounding chamber formed by the header shield. Therefore, the flow pattern of the hydrodynamic film may be directed away from the sterile interior and toward the spaces exterior to the rotating seals. Further, the chamber formed by the header shield may be provided with a steady supply of sterile air at a pressure lower than that in the annular space 710 but higher than ambient pressure. This "double redundancy" of the two surrounding sterile chambers at graduating pressures is theoretically analogous to that used in the design of clean rooms for sterile filing of pharmaceuticals.

The annular space 710 can be filled with a gas or liquid; preferably the gas or liquid is substantially sterile. The enclosed space thereby creates a barrier to microbes or other particulate materials passing into or out of the interior of the cell processing system. The gas or liquid may be introduced into the enclosed space via a channel which passes through or between the upper and lower elements. For example, referring to Fig. 31, air can be pumped through a 0.2 micron filter to assure sterility, and then be pumped through the gas port 634 to the channel 616 and into the annular space 710. Optionally the annular space 638 formed between the inner seal 700 and the first port 632 can be pressurized, for example when the annular space 638 is not conveying reagents into the processing container 604 or waste liquids out of the processing container. Preferably the annular space 710 is pressurized with sterile air at pressure slightly above atmospheric pressure. For example, it has been determined that an air pressure of approximately 0.25 PSIG suffices to provide a pressurized environment in the annular space to enhance the sealing function of the sealing members. Other pressures and gases can also be employed in similar fashion as will be evident to one of ordinary skill in the art. Alternatively, the annular space is pressurized relative to the exterior and interior of the rotating seal apparatus by evacuating the exterior and/or interior (e.g. the space inside the header shield and/or the annular space 638, respectively) by means of a vacuum pump or other device which creates a pressure differential.

The sterile air may be provided from a pressurized tank that uses a precise pressure regulating valve to reduce the tank pressure to a level that is slightly positive relative to ambient pressures (e.g. 0.25 PSIG). A computer software controlled "watchdog circuit" may be placed in communication with the annular space 710 to indicate, in a detectable manner, if and when undesirable pressure levels in the interior of the cell processing system occur. Furthermore, alterations in pressure in the annular space, detectable by a pressure monitor, may alert a system operator that one of the sealing elements has been breached and/or the barrier function of the annular space has been disrupted.

A second redundant sterile chamber can be created by the header shield assembly (shield top, shield bottom and shield clamp) that surrounds both the first and the second seal members. Sterile air may be supplied to this chamber at a pressure that is less than that of the annular space between the seals but greater than the surrounding ambient condition. The flow of air is from areas of higher pressure to areas of lower pressure. Therefore, the flow of sterile air may be directed from the inside of the seal and in an outward direction. Potential microbial contamination may thus be swept away from the sterile interior of the seal by this flow vector.

A serpentine seal 676 is formed between the close tolerance of the opposing flanges 672, 652 of the shield bottom and shield clamp. The serpentine seal may create shear forces between the surfaces of the flanges which prevent particulate material from the outside the shield from entering the shield and thus the seal assembly. In general, the serpentine seal acts as a physical barrier, if not a seal, to contaminants external to the rotating seal apparatus.

Additionally, the rotating seal apparatus can be formed of two concentric lip seals or two concentric barrel seals. Between lip seals or barrel seals is an annular space analogous to annular space 710. The annular space is in communication with a source of gas or liquid (*e.g.*, sterile pressurized air). Additional seal types will be known to one of ordinary skill in the art.

In operation, the rotating seal apparatus is provided as a preassembled device, properly preloaded by spring biasing force to create seals between the upper and lower sealing members. The rotating seal apparatus is placed in a cell processing system centrifugal device, for example by mounting it on a centrifuge chuck by the mounts 686. Closing the centrifugal device causes the compression of the spring to a third position which forces the seals into closer contact than the preload contact and maintains the close contact during rotation. The ports 632, 634 and 636 are joined to an appropriate set of tubes, optionally via connectors, to provide cells, processing materials, sterile air, etc. to the rotating seal apparatus and processing container. Upon rotation of the centrifugal device, the upper seal member, body, header shield top and bottom remain stationary and the lower seal member, base (and attached processing container), and header shield clamp rotate while the integrity of the seals are maintained.

The sealing surfaces of the upper and lower sealing members can be formed or fabricated of a variety of materials well known to one of ordinary skill in the art. Suitable materials include ceramics, carbon phenolic, graphite and graphite derivatives, lubricious plastic materials such as nylon, delrin, teflon, rulon, bronze and alloys thereof, stainless steel, carbon nitrites, etc. The sealing members can be manufactured as a single piece or as a separate sealing portions and support portions of the sealing member. The sealing members can be manufactured, for example, by injection molding or other method of fabrication, followed by making the sealing surfaces flat (e.g. by grinding or lapping) and polishing. The sealing members thus treated have sealing surfaces which when contacted essentially prevent fluid passage. Preferred materials include ceramics and carbon phenolic compounds.

Preferably the upper and lower sealing elements are formed of ceramic which is lapped and polished.

Other parts of the apparatus are constructed of various polymeric materials which preferably are FDA-approved for medical devices. The header shield top 660. the header shield bottom 650 and the header shield clamp 670 are preferably constructed of high impact polystyrene (HIPS), although any rigid plastic known to one of ordinary skill in the art which has some elastomeric properties can be used. The body 630 and the base 680 are preferably constructed of polycarbonate which provides good strength and stability. Other like materials can also be used.

The spring 640 is constructed of an elastomeric material, and preferably is constructed of a medium durometer silicon material such as thermoset silicon or liquid injection molding silicon. One also could use various rubber materials for the spring, preferably materials which are FDA approved for medical devices.

The present invention may be used in a variety of cell and cell element processing procedures, including collection and/or washing or red blood cells, platelets, lymphocytes granulocytes, monocytes, and stem cells (e.g., from peripheral blood bone marrow, or cord blood) as well as other methods such as viral inactivation. Preferably the cell processing system may be used in methods of enzymatically converting blood type.

## Claims

1. An optical sensor (58;200) for characterising a fluid being transferred in a sterile manner in a cell processing system, said optical sensor (58;200) comprising:
a light source (294;212) constructed and arranged to emit light directed toward a fluid; and
a light detector (296;216) constructed and arranged to detect light emitted from the source (294;212) and having interacted with the fluid;
**characterised in that** said optical sensor (58;200) further comprises a fluid distribution manifold (256) including several conduits (314,319,335) for transferring the fluid during processing, said fluid distribution manifold (256) comprising a cuvette (348) constructed as part of the manifold (256), which cuvette (348) is constructed and arranged to convey said fluid; and
a control circuit (80), said light source (294;212) and said light detector (296;216) being connected to said control circuit (80), said light source (294;212) being constructed to emit light of at least one selected wavelength, and said light detector (296;216) being arranged to detect light having interacted with the fluid in said cuvette (348), said control circuit (80) being adapted to characterise the fluid in said cuvette (348)based on the detected light.

2. An optical sensor (58;200) as claimed in claim 1, wherein said light source (294;212) and said light detector (296;216) are arranged in a transmission geometry.

3. An optical sensor (58;200) as claimed in claim 1 or claim 2, wherein said cuvette (348) is made of an optical material suitable for sterilization using gamma radiation.

4. An optical sensor (58;200) as claimed in claim 3, wherein said cuvette (348) and said fluid distribution manifold (256) are disposable.

5. An optical sensor (58;200) as claimed in any of claims 1 to 4, wherein said light source (294;212) and said light detector (296;216) are arranged to detect red blood cells.

6. A method for characterising a sterile fluid during processing of biological cells in a cell processing system, said method comprising:
emitting light generated by a light source (294;212) and directing said light towards a fluid to be **characterised**; and
detecting said light with a light detector (296;216), the light having interacted with the fluid;
**characterised by** distributing the fluid in a fluid distribution manifold (256) during operation of the cell processing system, which manifold (256) includes several conduits (314,319,335) for transferring sterile fluid during processing and a cuvette (348) permanently connected to at least one of the conduits (314,319,335), such that said distributing step includes conveying said fluid in said cuvette (348);
emitting light of at least one selected wavelength from said light source (294;212) which is connected to a control circuit(80);
detecting said light having interacted with the fluid flowing inside the cuvette (348), said light detector (296;216) being connected to said control circuit (80); and
characterising the fluid in the cuvette (348) by the control circuit (80) based on the detected light.

7. A method as claimed in claim 6, further comprising:
expressing a process chemical from a processing module (60);
transferring a flow of the expressed process chemical to a waste container (72) *via* conduits, at least one of which conduits is permanently connected to said cuvette (348); and
upon detecting the presence of biological cells during characterisation, redirecting the flow to a cell container.

8. A method as claimed in claim 7, wherein said redirecting step includes temporarily reversing the expressing performed by the processing module (60) to draw back the biological cells, and actuating a valve by the control circuit.

9. A method as claimed in any of claims 6 to 8, wherein said directing step includes transmitting said light through said cuvette (348) from the light source (294;212) to the light detector (296;216), and wherein said characterising includes detecting red blood cells in said fluid.

## Patentansprüche

1. Optischer Sensor (58; 200) zur Charakterisierung eines Fluids, das auf eine sterile Art in ein Zellaufarbeitungssystem transferiert wird, wobei der optische Sensor (58; 200) umfasst:
eine Lichtquelle (294; 212) konstruiert und angeordnet, um Licht zu emittieren, das in Richtung eines Fluids gerichtet ist; und
einen Lichtdetektor (296; 216) konstruiert und angeordnet, um Licht zu detektieren, das von der Quelle (294; 212) emittiert wird und mit dem Fluid interagiert hat;
**dadurch gekennzeichnet, dass** der optische Sensor (58; 200) weiter umfasst einen Fluidverteiler (256), der mehrere Leitungen (314, 319, 335) beinhaltet, um das Fluid während der Aufarbeitung zu transferieren, wobei dieser Fluidverteiler (256) eine Küvette (348) umfasst, konstruiert als Teil des Verteilers (256), wobei die Küvette (348) konstruiert und angeordnet ist, um das Fluid zu befördern; und
einen Steuerstromkreis (80), wobei die Lichtquelle (294; 212) und der Lichtdetektor (296; 216) mit dem Steuerstromkreis (80) verbunden sind, wobei die Lichtquelle (294; 212) konstruiert ist, um Licht von mindestens einer ausgewählten Wellenlänge zu emittieren, und wobei der Lichtdetektor (296; 216) angeordnet ist, um Licht, das mit dem Fluid in der Küvette (348) interagiert hat, zu detektieren, wobei der Steuerstromkreis (80) eingerichtet ist, um das Fluid in der Küvette (348), basierend auf dem detektierten Licht, zu charakterisieren.

2. Optischer Sensor (58; 200) nach Anspruch 1, wobei die Lichtquelle (294; 212) und der Lichtdetektor (296; 216) in einer Übertragungsgeometrie angeordnet sind.

3. Optischer Sensor (58; 200) nach Anspruch 1 oder Anspruch 2, wobei die Küvette (348) aus einem optischen Material hergestellt ist, das geeignet ist für die Sterilisation unter Verwendung von Gammastrahlung.

4. Optischer Sensor (58; 200) nach Anspruch 3, wobei die Küvette (348) und der Fluidverteiler (256) Einwegartikel sind.

5. Optischer Sensor (58; 200) nach einem der Ansprüche 1 bis 4, wobei die Lichtquelle (294; 212) und der Lichtdetektor (296; 216) angeordnet sind, um rote Blutkörperchen zu detektieren.

6. Verfahren zum Charakterisieren eines sterilen Fluids während der Aufarbeitung von biologischen Zellen in einem Zellaufarbeitungssystem, wobei dieses Verfahren umfasst:
Emittieren von Licht, erzeugt durch eine Lichtquelle (294; 212), und Richten des Lichts in Richtung eines zu charakterisierenden Fluids; und
Detektieren des Lichts mit einem Lichtdetekor (296; 216), wobei das Licht mit dem Fluid interagiert hat;
**gekennzeichnet durch** Verteilen des Fluids in einem Fluidverteiler (256) während des Betriebs des Zellaufarbeitungssystems, wobei der Verteiler (256) mehrere Leitungen (314, 319, 335) beinhaltet, um das sterile Fluid während der Aufarbeitung zu transferieren, und eine Küvette (348), die permanent mit mindestens einer der Leitungen (314, 319, 335) verbunden ist, so dass der Verteilungsschritt das Befördern des Fluids in die Küvette (348) beinhaltet;
Emittieren von Licht von mindestens einer ausgewählten Wellenlänge von der Lichtquelle (294; 212), die mit einem Steuerstromkreis (80) verbunden ist;
Detektieren dieses Lichts, das mit dem Fluid, das im Inneren der Küvette (348) fließt, interagiert hat, wobei der Lichtdetektor (296, 216) mit dem Steuerstromkreis (80) verbunden ist; und
Charakterisieren des Fluids in der Küvette (348) **durch** den Steuerstromkreis (80), basierend auf dem detektierten Licht.

7. Verfahren nach Anspruch 6, weiter umfassend:
Ausgeben einer Prozesschemikalie von einem aufarbeitenden Modul (60);
Transferieren eines Flusses der ausgegebenen Prozesschemikalie zu einem Abfallbehälter (72) über Leitungen, von denen mindestens eine permanent mit der Küvette (348) verbunden ist; und
Umleitung des Flusses zu einem Zellgefäß bei Detektierung der Anwesenheit von biologischen Zellen während der Charakterisierung.

8. Verfahren nach Anspruch 7, wobei der Umleitungsschritt vorübergehend die Umkehrung des Ausgebens, das durch das aufarbeitende Modul (60) ausgeführt wird, um die biologischen Zellen zurückzuziehen, und die Betätigung eines Ventils durch den Steuerstromkreis beinhaltet.

9. Verfahren nach einem der Ansprüche 6 bis 8, wobei der richtende Schritt die Leitung des Lichts durch die Küvette (348) von der Lichtquelle (294; 212) zu dem Lichtdetektor (296; 216) beinhaltet, und wobei die Charakterisierung das Detektieren von roten Blutkörperchen in dem Fluid beinhaltet.

## Revendications

1. Capteur optique (58 ; 200) pour caractériser un fluide en cours de transfert d'une manière stérile dans un système de traitement de cellules, ledit capteur optique (58 ; 200) comportant :
une source de lumière (294 ; 212) construite et agencée pour émettre de la lumière dirigée vers un fluide ; et
un détecteur de lumière (296 ; 216) construit et agencé pour détecter de la lumière émise depuis la source (294 ; 212) et ayant interagi avec le fluide ;
**caractérisé en ce que** ledit capteur optique (58 ; 200) comporte en outre un collecteur (256) de distribution de fluide comprenant plusieurs conduits (314, 319, 335) pour transférer le fluide en cours de traitement, ledit collecteur (256) de distribution de fluide comportant une cuvette (348) construite en tant que partie du collecteur (256), laquelle cuvette (348) est construite et agencée pour transporter ledit fluide ; et
un circuit de commande (80), ladite source de lumière (294 ; 212) et ledit détecteur de lumière (296 ; 216) étant connectés audit circuit de commande (80), ladite source de lumière (294 ; 212) étant construite de façon à émettre de la lumière d'au moins une longueur d'onde choisie, et ledit détecteur de lumière (296 ; 216) étant agencé de façon à détecter de la lumière ayant interagi avec le fluide dans ladite cuvette (348), ledit circuit de commande (80) étant conçu pour caractériser le fluide dans ladite cuvette (348) sur la base de la lumière détectée.

2. Capteur optique (58 ; 200) selon la revendication 1, dans lequel ladite source de lumière (294 ; 212) et ledit détecteur de lumière (296 ; 216) sont agencés dans une géométrie de transmission.

3. Capteur optique (58 ; 200) selon la revendication 1 ou la revendication 2, dans lequel ladite cuvette (348) est formée d'une matière optique apte à une stérilisation utilisant un rayonnement gamma.

4. Capteur optique (58 ; 200) selon la revendication 3, dans lequel ladite cuvette (348) et ledit collecteur (256) de distribution de fluide sont jetables.

5. Capteur optique (58 ; 200) selon l'une quelconque des revendications 1 à 4, dans lequel ladite source de lumière (294 ; 212) et ledit détecteur de lumière (296 ; 216) sont agencés de façon à détecter des cellules rouges du sang.

6. Procédé pour caractériser un fluide stérile pendant le traitement de cellules biologiques dans un système de traitement cellulaire, ledit procédé comprenant :
l'émission d'une lumière générée par une source de lumière (294 ; 212) et le fait de diriger ladite lumière vers un fluide devant être **caractérisé** ; et
la détection de ladite lumière avec un détecteur de lumière (296 ; 216), la lumière ayant interagi avec le fluide ;
**caractérisé par** la distribution du fluide dans un collecteur (256) de distribution de fluide pendant le fonctionnement du système de traitement cellulaire, lequel collecteur (256) comprend plusieurs conduits (314, 319, 335) destinés à transférer un fluide stérile pendant le traitement, et une cuvette (348) raccordée de façon permanente à au moins l'un des conduits (314, 319, 335), de façon que ladite étape de distribution comprenne le transport dudit fluide dans ladite cuvette (348) ;
l'émission d'une lumière d'au moins une longueur d'onde choisie depuis ladite source de lumière (294 ; 212) qui est connectée à un circuit de commande (80) ;
la détection de ladite lumière ayant interagi avec le fluide s'écoulant à l'intérieur de la cuvette (348), ledit détecteur de lumière (296 ; 216) étant connecté audit circuit de commande (80) ; et
la caractérisation du fluide dans la cuvette (348) par le circuit de commande (80) sur la base de la lumière détectée.

7. Procédé selon la revendication 6, comprenant en outre :
l'expression d'une substance chimique de traitement depuis un module de traitement (60) ;
le transfert d'un écoulement de la substance chimique de traitement exprimée vers un récipient à déchets (72) en passant par des conduits, au moins l'un des conduits est raccordé de façon permanente à ladite cuvette (348) ; et
lors de la détection de la présence de cellules biologiques pendant la caractérisation, le fait de rediriger l'écoulement vers un récipient à cellules.

8. Procédé selon la revendication 7, dans lequel ladite étape consistant à rediriger comprend l'inversion temporaire de l'expression effectuée par le module de traitement (60) pour ramener en arrière les cellules biologiques, et l'actionnement d'une soupape par le circuit de commande.

9. Procédé selon l'une quelconque des revendications 6 à 8, dans lequel ladite étape consistant à diriger comprend la transmission de ladite lumière à travers ladite cuvette (348) depuis la source de lumière (294 ; 212) vers le détecteur de lumière (296 ; 216), et dans lequel ladite caractérisation comprend la détection de cellules rouges du sang dans ledit fluide.
